# EUROPEAN PATENT SPECIFICATION

(11) **EP 4 293 358 B1**
(45) Date of publication and mention of the grant of the patent: **08.04.2026**
(21) Application number: 23189987.3
(22) Date of filing: 11.09.2018
(51) Int. Cl.: G01N 33/574

(54) **VOLATILE ORGANIC COMPOUNDS AS CANCER BIOMARKERS**
FLÜCHTIGE ORGANISCHE VERBINDUNGEN ALS KREBS BIOMARKER
COMPOSÉS ORGANIQUE VOLATILES UTILISÉ COMME BIOMARQUEURS DU CANCER

(30) Priority: 14.09.2017 GB 201714797
(43) Date of publication of application: 20.12.2023
(62) Divisional of application: 18773823.2
(73) Proprietor: IP2IPO Innovations Limited, London N1C 4AG (GB)
(72) Inventor: HANNA, George, Northwood (GB); MARKAR, Sheraz, London (GB)
(74) Representative: Hutter, Anton

(56) References cited:
- WO-A1-2017/137741
- NAVANEETHAN UDAYAKUMAR ET AL: "Volatile organic compounds in bile can diagnose malignant biliary strictures in the setting of pancreatic cancer: a preliminary observation", GASTROINTESTINAL ENDOSCOPY, ELSEVIER, NL, vol. 80, no. 6, 11 June 2014 (2014-06-11), pages 1038 - 1045, XP029096291, ISSN: 0016-5107, DOI: 10.1016/J.GIE.2014.04.016
- BRADLEY CONFER ET AL: "Pancreatic Adenocarcinoma Is Associated With a Unique Spectrum of Serum Volatile Organic Compounds That Distinguishes It From Chronic Pancreatitis and Healthy Controls", CANCER., vol. 148, no. 4, suppl. 1, 1 April 2015 (2015-04-01), US, pages S391, XP055517749, ISSN: 0008-543X, DOI: 10.1002/cncr.29041
- UDAYAKUMAR NAVANEETHAN ET AL: "Volatile Organic Compounds in Urine for Noninvasive Diagnosis of Malignant Biliary Strictures: A Pilot Study", DIGESTIVE DISEASES AND SCIENCES., vol. 60, no. 7, 24 February 2015 (2015-02-24), US, pages 2150 - 2157, XP055517752, ISSN: 0163-2116, DOI: 10.1007/s10620-015-3596-x
- FREDERIC ZENHAUSERN ET AL: "Solid phase micro-extraction (SPME) of volatile molecules in body fluids as a diagnostic measure for pancreatic cancer", CANCERRESEARCH, 1 April 2008 (2008-04-01), XP055517846, Retrieved from the Internet <URL:http://cancerres.aacrjournals.org/content/68/9_Supplement/4471> [retrieved on 20181022]

## Description

The present invention relates to biomarkers, and particularly although not exclusively, to novel biological markers for diagnosing various conditions, such as cancer. In particular, the invention relates to the use of these compounds as diagnostic and prognostic markers in assays for detecting cancer, such as pancreatic cancer, and corresponding methods of detection. The invention also relates to methods of determining the efficacy of treating these diseases with a therapeutic agent, and apparatus for carrying out the assays and methods. The assays are qualitative and/or quantitative, and are adaptable to large-scale screening and clinical trials.

Pancreatic cancer is estimated to cause over 40,000 deaths annually in the U.S. and was estimated to be the fourth largest contributor to overall cancer deaths in 2017 [1]. Only 15-20% of patients have potentially curable disease at the time of diagnosis [2,3]. Referrals for investigation of suspected pancreatic cancer from primary care depend on symptom recognition. Large primary care database studies and patient surveys indicate that patients with pancreatic cancer visit their general practitioner frequently in the months and years prior to diagnosis [4]. However, almost half of patients are still diagnosed as a result of an emergency presentation to hospital in the UK [5]. Current National Institute for Health and Care Excellence (NICE) referral guidelines to assess for pancreatic cancer include people aged 60 and over with weight loss and other symptoms [6]. Early symptoms are intermittent and overlap with other common benign conditions. The difficulty in symptom recognition is compounded by a lack of effective objective diagnostic methods that could be employed within general practice. The vast majority of biomarker studies have targeted high-risk groups such as hereditary pancreatitis, familial pancreatic cancer and intraductal papillary mucinous neoplasms. Translation of biomarkers into clinical use to date has failed for a variety of reasons, including failure to include appropriate controls such as chronic pancreatitis and failure to account for confounding factors such as biliary obstruction and diabetes [7,8].

When colorectal cancer (CRCa) is diagnosed at its earliest stage, more than 9 in 10 people with CRCa will survive their disease for five years or more, compared with less than 1 in 10 when diagnosed at the latest disease stage [1]. The utilization of bowel symptoms as the primary diagnostic basis for CRCa has been shown to have a very poor positive predictive value [2]. Risk of CRCa in symptomatic patients can be assessed by different investigations. Flexible sigmoidoscopy is the gold standard investigation but the large scale of its application has resource implications and its cost-effectiveness depends on the predictive values of different symptoms. Guaiac faecal occult blood test has good sensitivity of 87-98% in CRCa detection, but highly variable and often unsatisfactory specificity (13-79%), requiring the repetition of the test on multiple stool samples. To date, the faecal occult blood test is neither recommended nor available for use as an intermediate test [3-6]. The faecal immunochemical testing requires a single stool sampling. Four systems are fully automated, and provide a quantitative measure of haemoglobin, allowing selection of a threshold of positivity to fit specific circumstances. As a result the research data available on sensitivity and specificity for CRCa is based on small numbers of cancers. The data suggest that, depending on the selected threshold for positivity, the sensitivity for CRCa varies between 35% and 86% with specificity between 85% and 95% [5,6]. However, there are no data on the sensitivity of the newer quantitative test for early-stage cancers. The multi-target stool DNA test, when compared with the faecal immunochemical test in a large multicentre study, the test showed a better specificity (92 *vs.* 73%), but a lower sensitivity (90 *vs.* 96%) [7].

Navantheen et al., "Volatile organic compounds in bile can diagnose malignant biliary strictures in the setting of pancreatic cancer: a preliminary observation", Gastrointestinal Endoscopy, vol. 80, no. 6, 11 June 2014, pages 1038-1045, discloses a method of identifying potential VOCs in the bile of patients with malignant biliary strictures from pancreatic cancer. Bradley et al., "Pancreatic adenocarcinoma is associated with a unique spectrum of serum volatile organic compounds that distinguishes it from chronic pancreatitis and healthy controls", Cancer, vol. 148, no. 4, 1 April 2015, page S391, discloses a method of identifying VOCs that discriminate patients with pancreatic ductal adenocarcinoma from those with chronic pancreatitis and healthy controls. Navaneethan et al., "Volatile Organic Compounds in Urine for Noninvasive Diagnosis of Malignant Biliary Strictures: A Pilot Study", Digestive Diseases and Sciences, vol. 60, no. 7, 24 February 2015, pages 2150-2157, teaches a method of selected-ion flow-tube mass spectrometry to analyse the concentration of VOCs in urine samples to diagnose malignant biliary strictures. WO 2017/137741 discloses a method of diagnosing cancer, in which the levels of VOCs are determined in a urine sample obtained from the subject. Zenhausern et al., "Solid phase micro-extraction (SPME) of volatile molecules in body fluids as a diagnostic measure for pancreatic cancer", Cancer Research, 1 April 2008, discusses methodology using solid phase micro-extraction (SPME) in combination with gas chromatography and mass spectrometry (GC/MS), to identify prognostic and diagnostic volatile organic compounds related to pancreatic cancer.

An alternative approach for faecal-based tests is exhaled breath testing with the potential for high compliance because of the nature of the test and the possibility for testing more than one disease with different VOC discriminative signatures [8,9]. Researchers using gas chromatography mass spectrometry (GC-MS) have suggested the existence of a breath volatile organic compounds (VOCs) profile specific to CRCa [10]. GC-MS is a good technique for VOC identification, however it is semi-quantitative in nature, and thus limited in the ability of research findings to be reproduced by different research groups. Furthermore, there is a substantial analytical time for each sample, which does not naturally lend itself to high throughput analysis. Selected ion flow tube mass spectrometry (SIFT-MS) has the advantage of being quantitative and permits real-time analysis [11,12]. There has only been one previous study evaluating the breath profile of patients following curative surgical resection of colorectal cancer [13]. That study utilised GC-MS and suggested that following removal of the CRCa that there was a change in the exhaled breath VOC profile, further highlighting a potential association between tumour metabolism and VOC production. The authors also hypothesised these results may provide a rationale for using breath analysis in the follow-up of patients after surgery and monitoring of disease-free survival [13]. However, that study did not examine patients with evidence of CRCa recurrence. None of the previous studies have externally validated the initial discovery findings.

What is required is a reliable non-invasive marker to identify patients suffering from cancer, such as pancreatic cancer and/or colorectal cancer. A diagnostic method to identify those patients with pancreatic cancer and/or colorectal cancer would be of immense benefit to patients and would raise the possibility of early treatment and improved prognosis.

The inventors have now determined several biomarkers or so-called signature compounds as being indicative of pancreatic cancer and/or colorectal cancer.

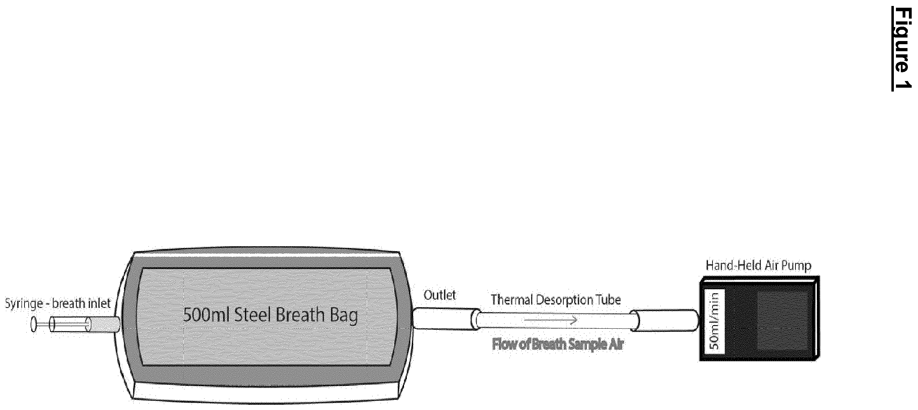

The inventors turned their attention first to diagnosis of pancreatic cancer. As described in Example 1, patients were recruited to two separate cohorts, an initial development study and second validation cohort. The cancer group included patients with localised and metastatic cancer, while the control group included patients with benign pancreatic disease or normal pancreas. The control test for comparison was radiological imaging with abdominal computerised tomography, ultrasound scan or endoscopic ultrasound confirmed with histo-pathological examination, as appropriate. Breath from the development cohort was collected with aluminium bags, and from the validation cohort using the ReCIVA system described in the Example and as shown in Figure 1. Analysis was performed using gas-chromatography mass-spectrometry. 68 patients were recruited to the development cohort (25 cancer, 43 non-cancer) and 64 to the validation cohort (32 cancer, 32 non-cancer). Of 66 signature volatile organic compounds (VOCs) identified, 13 were significantly different between groups on univariate analysis within the development cohort. Receiver operating characteristic analysis using significant volatiles and the validation cohort produced an area under the curve of 0.767 (sensitivity 81.3%, specificity 71.9%) differentiating cancer vs. non-cancer and 0.828 (sensitivity 77.8%, specificity 75%) differentiating adenocarcinoma and non-cancer.

The invention is as defined by the claims, and any other aspects, configurations, or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (of for diagnosis).

In a first aspect of the invention, there is provided a method for diagnosing a subject suffering from pancreatic cancer, or a pre-disposition thereto, or for providing a prognosis of the subject's condition, the method comprising analysing the concentration of a signature compound in a breath sample from a test subject and comparing this concentration with a reference for the concentration of the signature compound in an individual who does not suffer from pancreatic cancer, wherein a decrease in the concentration of the signature compound selected from a C₁-C₂₀ alkane in the breath sample from the test subject, compared to the reference, suggests that the subject is suffering from pancreatic cancer, or has a pre-disposition thereto, or provides a negative prognosis of the subject's condition.

In a second aspect of the invention, there is provided a method for determining the efficacy of treating a subject suffering from pancreatic cancer with a therapeutic agent or a specialised diet, the method comprising analysing the concentration of a signature compound in a breath sample from a test subject and comparing this concentration with a reference for the concentration of the signature compound in an individual who does not suffer from pancreatic cancer, wherein an increase in the concentration of the signature compound selected from a C₁-C₂₀ alkane in the breath sample from the test subject, compared to the reference, suggests that the treatment regime with the therapeutic agent or the specialised diet is effective, or wherein a decrease in the concentration of the signature compound selected from a C₁-C₂₀ alkane in the breath sample from the test subject, compared to the reference, suggests that the treatment regime with the therapeutic agent or the specialised diet is ineffective.

In a third aspect of the invention, there is provided the use of an apparatus for diagnosing a subject suffering from pancreatic cancer, or a pre-disposition thereto, or for providing a prognosis of the subject's condition, the apparatus comprising:-
(i) means for determining the concentration of a signature compound in a breath sample from a test subject; and
(ii) a reference for the concentration of the signature compound in a breath sample from an individual who does not suffer from pancreatic cancer,
wherein the apparatus is used to identify: a decrease in the concentration of the signature compound selected from a C₁-C₂₀ alkane in the breath sample from the test subject, compared to the reference, thereby suggesting that the subject suffers from pancreatic cancer, or has a pre-disposition thereto, or provides a negative prognosis of the subject's condition.

In a fourth aspect of the invention, there is provided the use of an apparatus for determining the efficacy of treating a subject suffering from pancreatic cancer with a therapeutic agent or a specialised diet, the apparatus comprising:-
(a) means for determining the concentration of a signature compound in a breath sample from a test subject; and
(b) a reference for the concentration of the signature compound in a breath sample from an individual who does not suffer from pancreatic cancer,
wherein the apparatus is used to identify:
(i) an increase in the concentration of the signature compound selected from a C₁-C₂₀ alkane in the breath sample from the test subject, compared to the reference, thereby suggesting that the treatment regime with the therapeutic agent or the specialised diet is effective; or
(ii) a decrease in the concentration of the signature compound selected from a C₁-C₂₀ alkane in the breath sample from the test subject, compared to the reference, thereby suggesting that the treatment regime with the therapeutic agent or the specialised diet is ineffective.

In a fifth aspect of the invention, there is provided the in vitro use of a signature compound selected from the group consisting of a C₁-C₂₀ alkane as a biomarker for diagnosing a subject suffering from pancreatic cancer, or a pre-disposition thereto, or for providing a prognosis of the subject's condition, wherein the signature compound is detected in a breath sample.

In an example, there is provided a method for diagnosing a subject suffering from pancreatic cancer, or a pre-disposition thereto, or for providing a prognosis of the subject's condition, the method comprising analysing the concentration of a signature compound in a bodily sample from a test subject and comparing this concentration with a reference for the concentration of the signature compound in an individual who does not suffer from pancreatic cancer, wherein (i) an increase in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, or (ii) a decrease in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, suggests that the subject is suffering from pancreatic cancer, or has a pre-disposition thereto, or provides a negative prognosis of the subject's condition.

In an example, there is provided a method for determining the efficacy of treating a subject suffering from pancreatic cancer with a therapeutic agent or a specialised diet, the method comprising analysing the concentration of a signature compound in a bodily sample from a test subject and comparing this concentration with a reference for the concentration of the signature compound in an individual who does not suffer from pancreatic cancer, wherein (i) a decrease in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, compared to the reference, or (ii) an increase in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, suggests that the treatment regime with the therapeutic agent or the specialised diet is effective, or wherein (i) an increase in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, compared to the reference, or (ii) a decrease in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, suggests that the treatment regime with the therapeutic agent or the specialised diet is ineffective.

In an example, there is provided an apparatus for diagnosing a subject suffering from pancreatic cancer, or a pre-disposition thereto, or for providing a prognosis of the subject's condition, the apparatus comprising:-
(i) means for determining the concentration of a signature compound in a sample from a test subject; and
(ii) a reference for the concentration of the signature compound in a sample from an individual who does not suffer from pancreatic cancer,
wherein the apparatus is used to identify: (i) an increase in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, or (ii) a decrease in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, thereby suggesting that the subject suffers from pancreatic cancer, or has a pre-disposition thereto, or provides a negative prognosis of the subject's condition.

In an example, there is provided an apparatus for determining the efficacy of treating a subject suffering from pancreatic cancer with a therapeutic agent or a specialised diet, the apparatus comprising:-
(a) means for determining the concentration of a signature compound in a sample from a test subject; and
(b) a reference for the concentration of the signature compound in a sample from an individual who does not suffer from pancreatic cancer,
wherein the apparatus is used to identify:
(i) a decrease in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, compared to the reference, or an increase in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, thereby suggesting that the treatment regime with the therapeutic agent or the specialised diet is effective; or
(ii) an increase in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, compared to the reference, or a decrease in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, thereby suggesting that the treatment regime with the therapeutic agent or the specialised diet is ineffective.

Methods of the disclosure may comprise administering or having administered, to the subject, a therapeutic agent or putting the subject on a specialised diet, wherein the therapeutic agent or the specialised diet prevents, reduces or delays progression of pancreatic cancer.

According to another example, there is provided a method of treating an individual suffering from pancreatic cancer, said method comprising the steps of:
(i) determining the concentration of a signature compound in a sample from a test subject concentration, wherein (i) an increase in the concentration of the signature compound selected from a C₁-C₃ aldehyde, C₁-C₃ alcohol, and C₂-C₁₀ alkane wherein a first carbon atom is substituted with the =O group and a second carbon atom is substituted with an -OH group, or an analogue or derivative thereof, in the bodily sample from the test subject, or (ii) a decrease in the concentration of the signature compound selected from a C₁-C₂₀ alkane, C₄-C₁₀ alcohol, C₁-C₆ carboxylic acid, and C₄-C₂₀ aldehyde, or an analogue or derivative thereof, in the bodily sample from the test subject, compared to the reference, suggests that the subject is suffering from pancreatic cancer, or has a pre-disposition thereto, or has a negative prognosis; and
(ii) administering or having administered, to the test subject, a therapeutic agent or putting the test subject on a specialised diet, wherein the therapeutic agent or the specialised diet prevents, reduces or delays progression of pancreatic cancer.

In another example, there is provided use of a signature compound selected from the group consisting of a C₁-C₃ aldehyde, C₁-C₃ alcohol, C₂-C₁₀ alkane wherein a first carbon atom is substituted with the =O group and a second carbon atom is substituted with an -OH group; C₁-C₂₀ alkane, C₄-C₁₀ alcohol, C₁-C₆ carboxylic acid, and C₄-C₂₀ aldehyde, or an analogue or derivative thereof, as a biomarker for diagnosing a subject suffering from pancreatic cancer, or a pre-disposition thereto, or for providing a prognosis of the subject's condition.

As described in Example 1, the inventors have shown that an increase in the concentration of formaldehyde, methanol, isopropyl alcohol or acetoin, or a decrease in the concentration of pentane, n-hexane, 1-butanol, propanoic acid, octanal, nonanal, decanal, undecanal, tetradecane, is indicative of pancreatic cancer. The methods, apparatus and uses described herein may also comprise analysing the concentration of an analogue or a derivative of the signature compounds described herein. Examples of suitable analogues or derivatives of chemical groups which may be assayed include alcohols, ketones, aromatics, organic acids and gases (such as CO, CO₂, NO, NO₂, H₂S, SO₂, CH₄).

In an example in which the signature compound is a C₁-C₃ aldehyde, preferably the compound is a C₁, C₂ or C₃ aldehyde, most preferably a C₁ aldehyde, i.e. formaldehyde.

In an example in which the signature compound is a C₁-C₃ alcohol, preferably the compound is a C₁, C₂ or C₃ alcohol, most preferably a C₁ alcohol (i.e. methanol) or a C₃ alcohol (i.e. isopropyl alcohol).

In an example in which the signature compound is a C₂-C₁₀ alkane wherein a first carbon atom is substituted with the =O group and a second carbon atom is substituted with an -OH group, preferably the compound is a C₁, C₂, C₃, C₄, C₅, C₆ C₇, C₈, C₉ or C₁₀ wherein a first carbon atom is substituted with the =O group and a second carbon atom is substituted with an -OH group. Preferably, the carbon atom substituted with the =O group is not a terminal carbon atom. More preferably, the compound is a C₃-C₆ alkane, and most preferably the compound is a C₄ alkane wherein a first carbon atom is substituted with the =O group and a second carbon atom is substituted with an -OH group, i.e. acetoin.

In an embodiment in which the signature compound is a C₁-C₂₀ alkane, preferably the compound is a C₁, C₂, C₃, C₄, C₅, C₆ C₇, C₈, C₉, C₁₀, C₁₁, C₁₂, C₁₃, C₁₄, C₁₅, C₁₆ C₁₇, C₁₈, C₁₉ or C₂₀ alkane, more preferably a C₃-C₁₅ alkane. It is preferred that the compound is a C₅-C₁₄ alcohol. For example, preferably the compound is a C₅ alcohol, i.e. pentane. Preferably, the compound is a C₆ alcohol, i.e. hexane. Preferably, the compound is a C₁₄ alcohol, i.e. tetradecane.

In an example in which the signature compound is a C₄-C₁₀ alcohol, preferably the compound is a C₄, C₅, C₆ C₇, C₈, C₉, C₁₀ alcohol. Preferably, the compound is a C₄-C₇ alcohol, most preferably a C₄ alcohol, i.e. butanol.

In an example in the signature compound is a C₁-C₆ carboxylic acid, preferably the compound is a C₁, C₂, C₃, C₄, C₅, C₆ carboxylic acid. Preferably, the compound is a C₂-C₄ carboxylic acid, more preferably a C₃ carboxylic acid, i.e. propanoic acid.

In an example in the signature compound is a C₄-C₂₀ aldehyde, preferably the compound is a C₄, C₅, C₆ C₇, C₈, C₉, C₁₀, C₁₁, C₁₂, C₁₃, C₁₄, C₁₅, C₁₆ C₁₇, C₁₈, C₁₉ or C₂₀ aldehyde. Preferably, the compound is a C₅-C₁₅ aldehyde, more preferably a C₇-C₁₃ aldehyde. Preferably, the compound is a C₈ aldehyde, i.e. octanal. Preferably, the compound is a C₉ aldehyde, i.e. nonanal. Preferably, the compound is a C₁₀ aldehyde, i.e. detanal. Preferably, the compound is a C₁₁ aldehyde, i.e. undecanal.

Thus, in an example, there is provided a method for diagnosing a subject suffering from pancreatic cancer, or a pre-disposition thereto, or for providing a prognosis of the subject's condition, the method comprising analysing the concentration of a signature compound in a bodily sample from a test subject and comparing this concentration with a reference for the concentration of the signature compound in an individual who does not suffer from pancreatic cancer, wherein (i) an increase in the concentration of the signature compound selected from formaldehyde, methanol, isopropyl alcohol and acetoin, or an analogue or derivative thereof, in the bodily sample from the test subject, or (ii) a decrease in the concentration of the signature compound selected from pentane, hexane, butanol, propanoic acid, octanal, nonanal, decanal, undecanal, tetradecane, or an analogue or derivative thereof, in the bodily sample from the test subject, compared to the reference, suggests that the subject is suffering from pancreatic cancer, or has a pre-disposition thereto, or provides a negative prognosis of the subject's condition.

It will be appreciated that the examples also involve detecting the same signature compounds as in the previous paragraph.

The inventors then turned their attention to diagnosis of colorectal cancer. As described in Example 2, exhaled breath samples were collected using 2-Litre double-layered Nalophan bags, and were analyzed using Selected-Ion-Flow-Tube Mass-Spectrometry. Gold-standard test for comparison was endoscopy for luminal inspection and CT to confirm cancer recurrence. Three studies were conducted: (i) profiling study: 150 patients; 50 CRCa and 100 controls; (ii)i diagnostic validation: 79 patients; 25 CRCa and 54 controls; and (iii) clinical validation with tumour recurrence: 40 patients; 19 postoperative (no recurrence) and 21 CRCa recurrences. In multivariate analysis, a single VOC, propanal, was significantly elevated in the cancer cohort compared with control patients. Using a threshold of 28ppbv this gave a sensitivity of 96% and specificity of 76% for CRCa diagnosis. Propanal was similarly elevated with CRCa and using a threshold of 28ppbv this gave a sensitivity of 83.3% and specificity of 84.7%. Following surgery, propanal reduced to levels expected in control patients, and with recurrence, levels increased significantly. Using a threshold of 28ppbv the sensitivity for identification of CRCa recurrence was 71.4% and specificity was 90.9%.

As described in Example 2, the inventors have shown that an increase in propanal is indicative of colorectal cancer. The methods, apparatus and uses described herein may also comprise analysing the concentration of an analogue or a derivative of the signature compounds described herein. Examples of suitable analogues or derivatives of chemical groups which may be assayed include alcohols, ketones, aromatics, organic acids and gases (such as CO, CO₂, NO, NO₂, H₂S, SO₂, CH₄).

An important feature of any useful biomarker used in disease diagnosis and prognosis is that it exhibits high sensitivity and specificity for a given disease. As explained in the examples, the inventors have surprisingly demonstrated that a number of signature compounds found in the exhaled breath from test subjects serve as robust biomarkers for diseases, such as pancreatic cancer and colorectal cancer, and can therefore be used for the detection of these diseases, and disease prognosis. In addition, the inventors have shown that using such signature compounds as a biomarker for disease employs an assay which is simple, reproducible, non-invasive and inexpensive, and with minimal inconvenience to the patient.

Advantageously, the methods and apparatus of the invention provide a non-invasive means for diagnosing various cancers. The methods according to this disclosure are useful for enabling a clinician to make decisions with regards to the best course of treatment for a subject who is currently or who may suffer from pancreatic cancer or colorectal cancer, respectively. It is preferred that the methods of this disclosure are useful for enabling a clinician to decide how to treat a subject who is currently suffering from the cancer. In addition, the methods of this disclosure are useful for monitoring the efficacy of a putative treatment for the relevant cancer. For example, if the cancer is pancreatic cancer, then treatment may comprise administration of chemotherapy, chemoradiotherapy with or without surgery. For example, if the cancer is colorectal cancer, then treatment may comprise administration of chemotherapy, chemoradiotherapy with or without surgery, or endoscopic resection.

Hence, the apparatus according to this disclosure are useful for providing a prognosis of the subject's condition, such that the clinician can carry out the treatment according to this disclosure. The apparatus of this disclosure may be used to monitor the efficacy of a putative treatment for the cancer. The methods and apparatus are therefore very useful for guiding a treatment regime for the clinician, and to monitor the efficacy of such a treatment regime. The clinician may use the apparatus of the invention in conjunction with existing diagnostic tests to improve the accuracy of diagnosis.

The subject may be any animal of veterinary interest, for instance, a cat, dog, horse etc. However, it is preferred that the subject is a mammal, such as a human, either male or female.

Preferably, a sample is taken from the subject, and the concentration of the signature compound in the bodily sample is then measured.

The signature compounds, which are detected, are known as volatile organic compounds (VOCs), which lead to a fermentation profile, and they may be detected in the bodily sample by a variety of techniques. In one embodiment, these compounds may be detected within a liquid or semi-solid sample in which they are dissolved. In a preferred embodiment, however, the compounds are detected from gases or vapours. For example, as the signature compounds are VOCs, they may emanate from, or form part of, the sample, and may thus be detected in gaseous or vapour form.

The apparatus of this disclosure may comprise sample extraction means for obtaining the sample from the test subject. The sample extraction means may comprise a needle or syringe or the like. The apparatus may comprise a sample collection container for receiving the extracted sample, which may be liquid, gaseous or semi-solid.

Preferably, the sample is any bodily sample into which the signature compound is present or secreted. For example, the sample may comprise urine, faeces, hair, sweat, saliva, blood or tears. The inventors believe that the VOCs are breakdown products of other compounds found within the blood. In one example, blood samples may be assayed for the signature compound's levels immediately. Alternatively, the blood may be stored at low temperatures, for example in a fridge or even frozen before the concentration of signature compound is determined. Measurement of the signature compound in the bodily sample may be made on whole blood or processed blood.

In other example, the sample may be a urine sample. It is preferred that the concentration of the signature compound in the bodily sample is measured *in vitro* from a urine sample taken from the subject. The compound may be detected from gases or vapours emanating from the urine sample. It will be appreciated that detection of the compound in the gas phase emitted from urine is preferred.

It will also be appreciated that "fresh" bodily samples may be analysed immediately after they have been taken from a subject. Alternatively, the samples may be frozen and stored. The sample may then be de-frosted and analysed at a later date.

Most preferably, however, the bodily sample may be a breath sample from the test subject. The sample may be collected by the subject performing exhalation through the mouth, preferably after nasal inhalation. Preferably, the sample comprises the subject's alveolar air. Preferably, the alveolar air was collected over dead space air by capturing end-expiratory breath. VOCs from breath bags were then preferably pre-concentrated onto thermal desorption tubes by transferring breath across the tubes.

The difference in concentration of signature compound in the methods or the apparatus of this disclosure may be an increase or a decrease compared to the reference. As described in the examples, the inventors monitored the concentration of the signature compounds in numerous patients who suffered from either pancreatic or colorectal cancer, and compared them to the concentration of these same compounds in individuals who did not suffer from the disease (i.e. reference or controls). They demonstrated that there was a statistically significant increase or decrease in the concentration of these compounds in the patients suffering from the disease.

It will be appreciated that the concentration of signature compound in patients suffering from a disease is highly dependent on a number of factors, for example how far the disease has progressed, and the age and gender of the subject. It will also be appreciated that the reference concentration of signature compound in individuals who do not suffer from the disease may fluctuate to some degree, but that on average over a given period of time, the concentration tends to be substantially constant. In addition, it should be appreciated that the concentration of signature compound in one group of individuals who suffer from a disease may be different to the concentration of that compound in another group of individuals who do not suffer from the disease. However, it is possible to determine the average concentration of signature compound in individuals who do not suffer from the cancer, and this is referred to as the reference or 'normal' concentration of signature compound. The normal concentration corresponds to the reference values discussed above.

In one embodiment, the methods of the invention preferably comprise determining the ratio of chemicals within the breath (i.e. use other components within it as a reference), and then compare these markers to the disease to show if they are elevated or reduced.

The signature compound is preferably a volatile organic compound (VOC), which leads to a fermentation profile, and it may be detected in or from the bodily sample by a variety of techniques. Thus, these compounds may be detected using a gas analyser. Examples of suitable detector for detecting the signature compound preferably includes an electrochemical sensor, a semiconducting metal oxide sensor, a quartz crystal microbalance sensor, an optical dye sensor, a fluorescence sensor, a conducting polymer sensor, a composite polymer sensor, or optical spectrometry.

The inventors have demonstrated that the signature compounds can be reliably detected using gas chromatography, mass spectrometry, GCMS or TOF. Dedicated sensors could be used for the detection step.

The reference values may be obtained by assaying a statistically significant number of control samples (i.e. samples from subjects who do not suffer from the disease). Accordingly, the reference (ii) according to the apparatus of this disclosure may be a control sample (for assaying).

The apparatus preferably comprises a positive control (most preferably provided in a container), which corresponds to the signature compound(s). The apparatus preferably comprises a negative control (preferably provided in a container). In a preferred embodiment, the apparatus may comprise the reference, a positive control and a negative control. The apparatus may also comprise further controls, as necessary, such as "spike-in" controls to provide a reference for concentration, and further positive controls for each of the signature compounds, or an analogue or derivative thereof.

Accordingly, the inventors have realised that the difference in concentrations of the signature compound between the reference normal (i.e. control) and increased/decreased levels, can be used as a physiological marker, suggestive of the presence of a disease in the test subject. It will be appreciated that if a subject has an increased/decrease concentration of one or more signature compounds which is considerably higher/lower than the 'normal' concentration of that compound in the reference, control value, then they would be at a higher risk of having the disease, or a condition that was more advanced, than if the concentration of that compound was only marginally higher/lower than the 'normal' concentration.

The inventors have noted that the concentration of signature compounds referred to herein in the test individuals was statistically more than the reference concentration (as calculated using the method described in the Example). This may be referred to herein as the 'increased' concentration of the signature compound.

The skilled technician will appreciate how to measure the concentrations of the signature compound in a statistically significant number of control individuals, and the concentration of compound in the test subject, and then use these respective figures to determine whether the test subject has a statistically significant increase/decrease in the compound's concentration, and therefore infer whether that subject is suffering from the disease which has been screened for.

In the method of and the apparatus of this disclosure, the difference in the concentration of the signature compound in the bodily sample compared to the corresponding concentration in the reference is indicative of the efficacy of treating the subject's disease with the therapeutic agent, and surgical resection. The difference may be an increase or a decrease in the concentration of the signature compound in the bodily sample compared to the reference value. In examples where the concentration of the compound in the bodily sample is lower than the corresponding concentration in the reference, then this would indicate that the therapeutic agent or specialist diet is successfully treating the disorder in the test subject. Conversely, where the concentration of the signature compound in the bodily sample is higher than the corresponding concentration in the reference, then this would indicate that the therapeutic agent or specialist diet is not successfully treating the disorder.

All features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
**Figure 1** shows an embodiment of an apparatus and a method used for concentrating VOCs from steel breath bags onto thermal desorption tubes;
**Figure 2** shows ROC plots of sensitivity against 1-specificity produced for A) Cancer vs. Non-Cancer and B) All adenocarcinoma vs. Non-Cancer using data from the development cohort (Bags). The Tables below summarize ROC analysis data including area under the curve (AUC);
**Figure 3** shows ROC plots of sensitivity against 1-specificity produced for A) Cancer vs. Non-Cancer and B) All adenocarcinoma vs. Non-Cancer using data from the validation cohort (ReCIVA). The Tables below summarize ROC analysis data including area under the curve (AUC);
**Figure 4** shows the results of Study (i) - profiling diagnostic investigation; ROC analysis for propanal as a diagnostic marker of colorectal cancer in comparison with negative control patients (AUC = 0.90 ± 0.03, 95% CI 0.83-0.96);

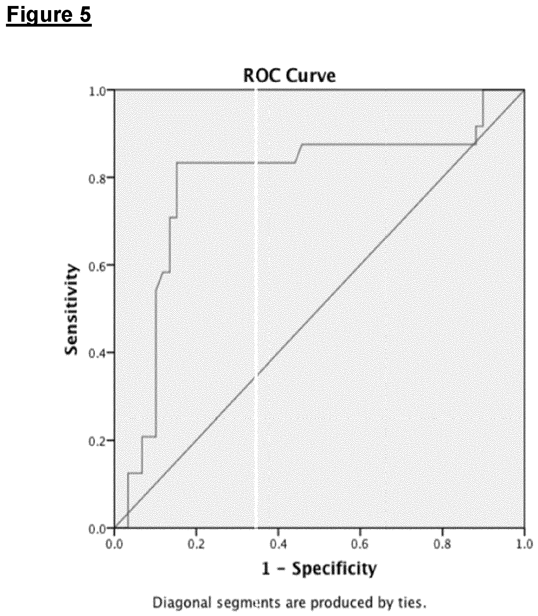

**Figure 5** shows the results of Study (ii) - independent diagnostic validation; ROC analysis of independent validation study of propanal for the diagnosis of Colorectal cancer (AUC = 0.79 ± 0.06, 95%CI 0.66-0.91);

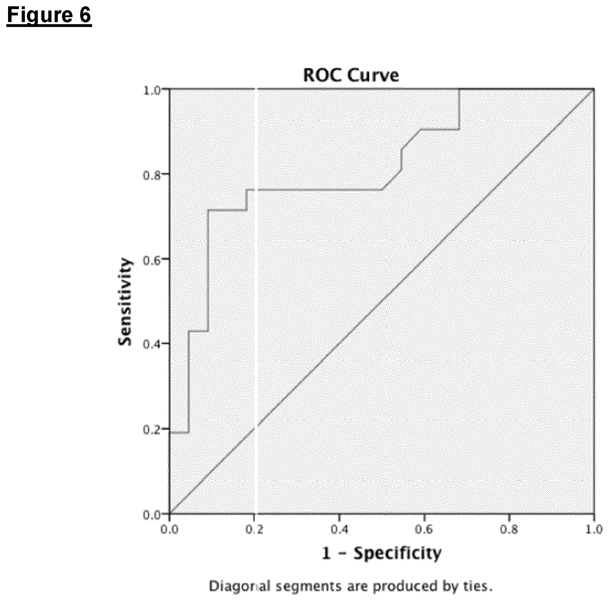

**Figure 6** shows the results of Study (iii) - clinical validation with tumor recurrence; propanal upregulation associated with the presence of CRCa recurrence following primary CRCa surgical resection (AUC = 0.81 ± 0.07, 95%CI 0.68-0.94); and

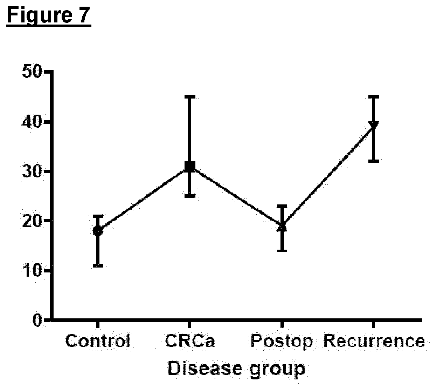

**Figure 7** shows the changes observed in average propanal concentration across the three studies and four disease states studied.

### Examples

The inventors investigated the use of VOCs for detecting a range of different cancers, including pancreatic and colorectal cancer.

### Example 1 - Pancreatic cancer

Pancreatic cancer has a very poor prognosis as most patients are diagnosed at an advanced stage when curative treatments are not possible. Breath volatile organic compounds have shown potential as novel biomarkers to detect other cancers types. This study identified and validated a unique breath volatile organic compound profile associated with the presence of pancreatic cancer, suggesting the potential of breath analysis for inclusion in the pancreatic cancer diagnostic pathway.

The primary objective of this study was to profile changes observed in the exhaled breath VOCs using Thermal Desorption Gas Chromatography Mass Spectrometry (TD-GC-MS) for patients with primary pancreatic cancer, positive control disease and normal pancreas. The secondary objective was to develop diagnostic models for the identification of pancreatic neoplasms and specifically adenocarcinoma, with further validation in an independently collected second cohort of patients. The third objective was to quantify differences in volatile organic compounds in the exhaled breath of pancreatic cancer compared to non-cancer cohorts and to generate cancer diagnostic models.

### Materials and Methods

Two studies were conducted. In the first profiling study, exhaled breath was collected and analyzed to identify VOCs that differed in concentration between the cancer and control patients. Those compounds were used to develop the diagnostic model for pancreatic cancer. This model was then validated using a second independently collected cohort of patients.

### Study Population

All enrolled patients were recruited from the Imperial College NHS trust from March 2016 to December 2016. Regional ethical approval for was granted (REC ref: 14/LO/1136). The details of the study were explained to all eligible patients and fully informed and written consent was obtained prior to enrolment. Demographic and clinical information were collected.

In both the profiling and validation studies, pancreatic cancer patients were compared with a control groups that include benign pancreatic diseases. For the pancreatic cancer group, patients with localised pancreatic cancer were sampled pre-operatively on surgical wards or from the endoscopy unit prior to undergoing endoscopic ultrasound.

Patients with metastatic pancreatic disease were recruited from oncology clinics. For the control group, patients were recruited with a diagnosis of other pancreatic conditions including; intra-ductal papillary mucinous neoplasm (IPMN), cysts, pseudocysts and chronic pancreatitis. Patients scheduled for elective upper abdominal ultrasound (US) with a normally appearing pancreas on imaging were recruited to this group.

### Reference test

All cases were confirmed with a standard reference test. Pancreatic cancer was confirmed by abdominal Computerized Tomography (CT) or endoscopic ultrasound and histologically by fine-needle aspirate biopsy. Abdominal CT or ultrasound examined the pancreas of patients within the control group.

### Exhaled breath collection

Exhaled breath collection was performed using a previously validated methodology [11] that was informed by the inventors' investigations on the influence of breath manoeuvres and hospital environment on VOC measurements [17,18]. All patients were fasted for a minimum of 4 hours prior to breath sampling to minimise the risk of oral contamination or dietary intake acting as a confounder. Atmospheric air from sample collection rooms and the laboratory were also analysed to investigate the effects of background VOCs on collected breath samples. The method of breath sampling was changed from inert aluminium bags (Bedfont Scientific Ltd., Maidstone, UK) in the initial profiling study to ReCIVA breath sample system (Owlstone Medical Inc., Cambridge, UK) in the validation study.

Referring to Figure 1, there is shown an ReCIVA apparatus used for the breath sampling in accordance with the invention. The ReCIVA apparatus is a reproducible system that allows direct breath collected into the thermal desorption tubes, which is the system to be used in future multi-centre studies.

Breath was collected using 500ml inert aluminium bags that were washed through with synthetic air prior to sampling. Patients were asked to perform deep nasal inhalation followed by complete exhalation through the mouth. Alveolar air was preferentially collected over dead space air by capturing end-expiratory breath. VOCs from breath bags were then pre-concentrated (see Figure 1) onto thermal desorption tubes by transferring 250ml of breath at 50ml/sec across the tubes with 10mm diameter tubing and hand-held air pumps (210-1002MTX, SKC ltd., Dorset, UK).

For the ReCIVA system, breath sampling remains completely non-invasive and involves placing a disposable facemask around the nose and mouth of the patient and instructing them to perform normal tidal breathing. A constant supply of air is ventilated to the patient's mask by the Capser system (Owlstone Medical Inc., Cambridge, UK), ensuring that the patient inspires only clean air. The ReCIVA apparatus uses an internal CO2 monitor and pressure sensors to preferentially capture alveolar breath and transfer it directly onto thermal desorption tubes. Similarly to bag collection, a total of 250mls of alveolar breath was transferred onto the thermal desorption tubes.

### Mass Spectrometric Analysis

All air samples were analysed within 48 hours of collection. Data from degradation studies have shown that volatiles remain stable within breath bags for 48 hours [19]. TD-GC-MS is an analytical method used for the identification and quantification of volatile and semi-volatile compounds. The VOCs entering the device travel through the chromatography column (7890B GC, Agilent technologies, Cheadale, UK), are separated according to their affinity with the stationary phase, and leave the column at a specific retention time. Then, VOCs enter a mass spectrometer (5977A MSD, Agilent technologies, UK), where they are ionised, accelerated, deflected and detected based upon their mass/charge (m/z) ratios. The combination of both gas chromatography and mass spectrometry allows for improved compound identification than the use of either component individually.

Thermal desorption tubes were used to concentrate volatiles prior to GC-MS analysis by fixing them to Tenax sorbent that line the inside of the tube. All Tenax TA tubes (Markes International, UK) were conditioned (TC-20, Markes International, UK) at 300°C for 1 hour 10 minutes. The tubes were loaded onto carousels, checked for tube leaks and then dry purged for 3 minutes to remove excess moisture as to ensure that VOCs were not oxidised upon heating. The tubes then underwent desorption (TD-100, Markes International, UK) at 280°C onto a 10°C cold trap for 10 minutes (nitrogen flow 50ml/min). The cold trap was then rapidly heated to 290°C transferring the VOCs to the chromatography column. In an attempt to minimise background VOCs fixed to the tubes, the time from tube conditioning to pre-concentration never exceeded one hour.

### GC-MS methodology

The initial oven temperature was held at 40°C for 4 min, then ramped 5°C/min to 100°C with a 1-minute hold, ramped 5°C/min to 110°C with a 1-minute hold, ramped 5°C/min to 200°C, and finally ramped 10°C/min to 240°C with a 4-minute hold. The total GC analysis time was 47 minutes. The mass spectrometer was operated with the electron impact ionisation mode, scanning mass ions 20-250 m/z at 5.9 scans/sec. Temperature of the mass spectrometers quadrupole and source were 230°C and 150°C respectively. A solvent delay of 3 minutes was used at the start of the run to minimize interference from water.

### Data Extraction

Chromatograms and mass spectra data were extracted onto a qualitative analysis software (Agilent Masshunter Qualitative Analysis, UK). The chemical identity of every peak, with retention times between 3-47 minutes, was then confirmed with the NIST database. The retention time and characteristic m/z ion from identified VOCs were used for the quantification of their abundance (Agilent Masshunter Quantitative Analysis, UK) across all chromatograms. A retention time range of ±0.1 minutes was used in the quantification, ensuring that only characteristic ions from a 0.2-minute range were quantified.

### Statistical Analysis

All statistical analysis was performed using IBM SPSS 24 (IBM corp., Armonk, NY). P values less than 0.05 were considered significant, and all statistical tests were two-sided. Cancer disease status and confounding factors were considered independent variables and VOC abundance was considered the dependent variable. A Shapiro-Wilk statistical test was performed.

Significant differences in the abundance of volatiles between cancer and non-cancer groups in the development cohort were assessed using univariate Mann-Whitney U statistical tests (as data was non-normally distributed). VOCs found to be significant on univariate analysis were included in logistic regression analysis to form the basis of a diagnostic model for use in the validation cohort. Receiver Operating Characteristic (ROC) plots were produced by plotting the true positive rate (sensitivity) against the false positive rate (1-specificity). Two ROC plots were produced for cancer vs. non-cancer and adenocarcinoma vs. non-cancer comparisons. The Area Under the Curve (AUC) was used to assess the prediction power of the model and its ability to distinguish between cancer and non-cancer. Sensitivity and specificity values were extracted from the coordinates of the ROC plots. The cancer group included all subgroups of pancreatic cancer while the non-cancer group included both positive control and normal pancreas groups. The adenocarcinoma group consisted of both localised and metastatic adenocarcinoma subgroups.

Statistical analysis was also performed to identify significant differences between the groups in age, ethnicity, sex, gastroesophageal reflux disease (GERD), pancreatitis, hepatic disease, hepatitis, diabetes mellitus, smoking status and alcohol intake. Kruskal-Wallis was employed for continuous age data, while all other nominal potential confounder data was assessed using Chi-squared/Fisher exact test/likelihood ratio depending on the expected count numbers and the number of variables tested. All confounders were subsequently tested against VOC abundance with linear regression.

### Results

### Patients

A total of 68 patients (see Table 1) were recruited to the model development cohort. Patients were assigned to cancer (n=25) and non-cancer (n=43) groups, including localised adenocarcinoma (n=7), localised neuroendocrine tumour (NET) (n=4), metastatic adenocarcinoma (n=10), metastatic NET (n=4), positive control (n=20), and normal pancreas (n=23).

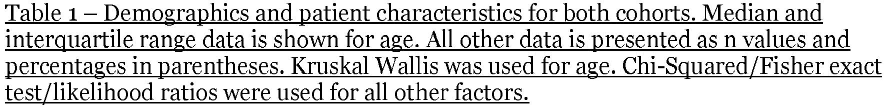

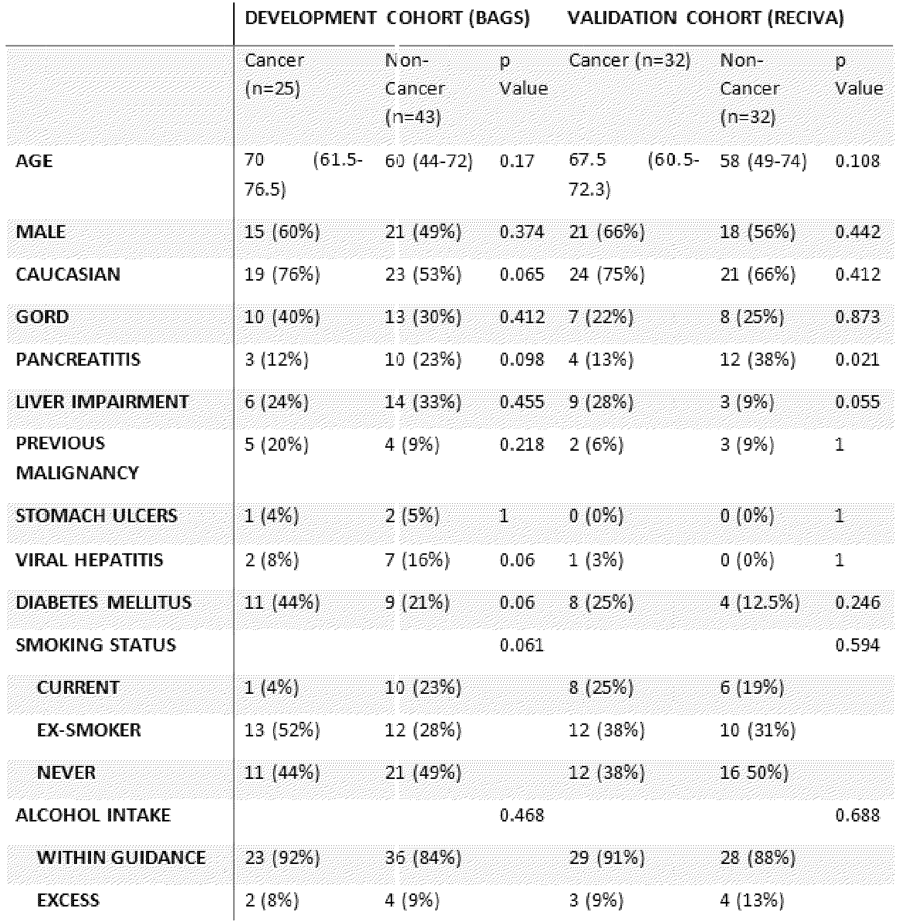

A further 64 patients were recruited to the validation cohort. Patients were again divided into cancer (n=32) and non-cancer (n=32) groups, and included local adenocarcinoma (n=14), local NET (2), metastatic adenocarcinoma (14), metastatic NET (3), positive control (24), and normal pancreas (8). There were no significant differences in patient demographics or comorbidities between the cancer and control groups (see Table 1).

### VOC Analysis

Qualitative analysis of chromatograms yielded 66 VOCs that were identifiable from the NIST database. Twenty-two of these VOCs were excluded from further analysis as they were either found to be in high concentrations in background air or considered unlikely to be endogenously produced. The identity of the remaining 44 VOCs, as well as their retention times and characteristic m/z ratio, were subsequently used for quantification of VOC abundance.

Shapiro-Wilk testing revealed that abundance data for all VOCs were not normally distributed. Univariate Mann Whitney U tests revealed 9 VOCs (Table 2) with significantly altered abundances in cancer within the development cohort (formaldehyde, methanol, pentane, isopropyl alcohol, n-hexane, acetoin, octanal, undecanal, tetradecane).

**Table 2 - Table of 11 significant VOCs. Arrows indicate the direction of change for the cancer cohort. Arrows are omitted for non-significant VOCs. P values produced from Mann Whitney U tests for both Cancer Vs Non-Cancer and Adenocarcinoma Vs Non-Cancer**

| **VOCs** | **Cancer vs Non-Cancer** | | | **Adenocarcinoma vs Non-Cancer** | | |
|---|---|---|---|---|---|---|
| | Bags | ReCIVA | p value | Bags | ReCIVA | p value |
| Formaldehyde | ↑ | ↑ | 0.004 | ↑ | ↑ | 0.022 |
| Methanol | ↑ | ↑ | 0.002 | ↑ | ↑ | 0.004 |
| Pentane | ↓ | ↓ | 0.002 | ↓ | ↓ | 0.007 |
| Isopropyl Alcohol | ↑ | ↑ | 0.001 | ↑ | ↑ | 0.001 |
| n-Hexane | ↓ | ↓ | <0.001 | ↓ | ↓ | 0.001 |
| 1-Butanol | | | 0.3 | ↓ | ↓ | 0.005 |
| Acetoin | ↑ | ↑ | 0.001 | ↑ | ↑ | <0.001 |
| Propanoic Acid | | | 0.07 | ↓ | ↓ | 0.008 |
| Octanal | ↓ | ↓ | 0.028 | ↓ | ↓ | 0.041 |
| Nonanal | | | 0.434 | ↓ | ↓ | 0.016 |
| Decanal | | | 0.188 | ↓ | ↓ | 0.038 |
| Undecanal | ↓ | ↓ | 0.047 | | | 0.067 |
| Tetradecane | ↓ | ↓ | 0.017 | | | 0.259 |

Further analysis also revealed 11 VOCs (Table 2) with significantly altered abundances in an adenocarcinoma vs non-cancer comparison (formaldehyde, methanol, pentane, isopropyl alcohol, n-hexane, 1-butanol, acetoin, propanoic acid, octanal, nonanal, decanal).

Of these significant VOCs, the abundances of 4 were found to be raised in cancer (formaldehyde, methanol, isopropyl alcohol, acetoin), and the remaining 9 were found to be reduced in cancer breath (pentane, n-hexane, 1-butanol, propanoic acid, octanal, nonanal, decanal, undecanal, tetradecane) (Table 2). This direction of change was found to be the same for all significant VOCs in data from both the development and validation cohorts.

Linear regression analysis (Table 3) revealed pancreatic cancer disease status was the strongest predictor for all significant VOC abundances. No confounders were found to be independent predictors of abundance of any of the significant VOCs.

### Receiver Operator Characteristic (ROC) Analysis

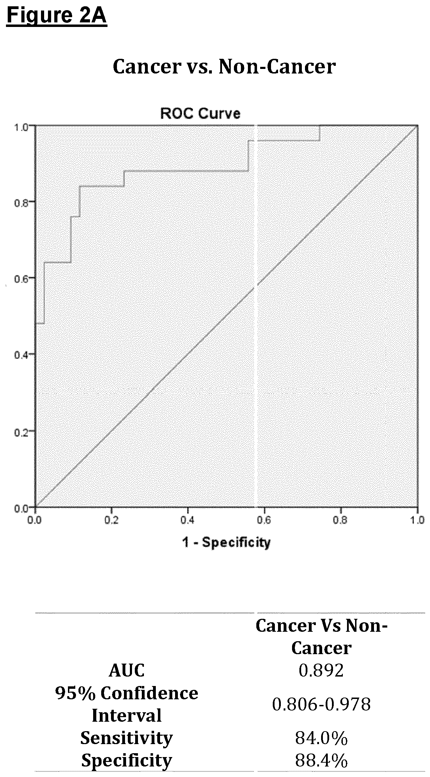

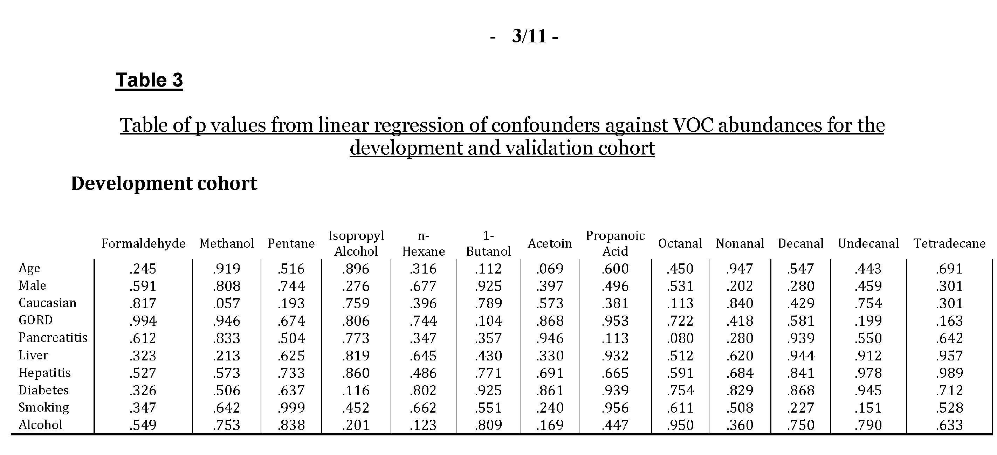

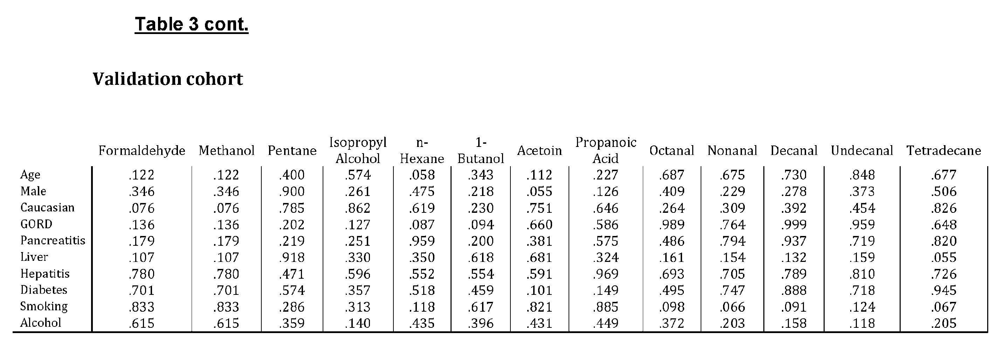

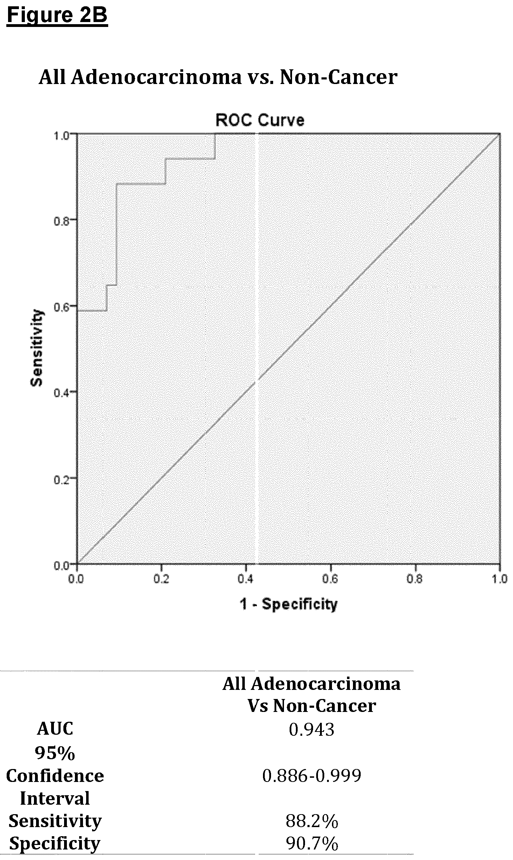

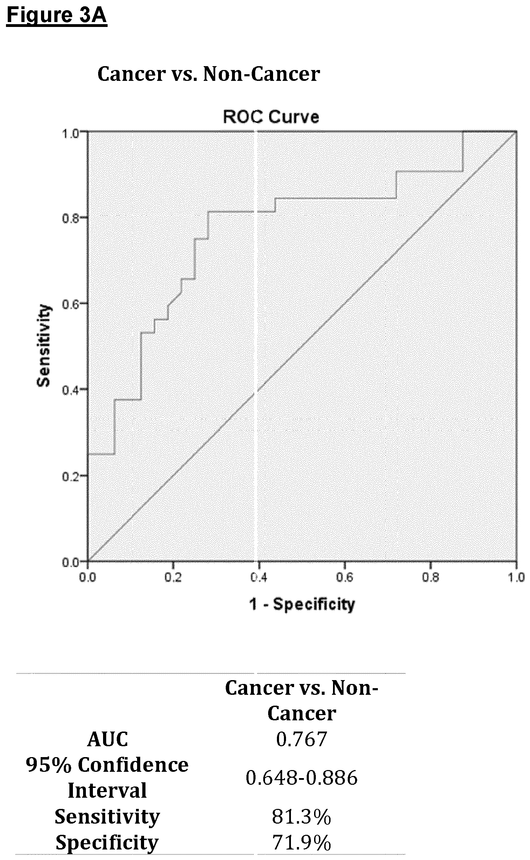

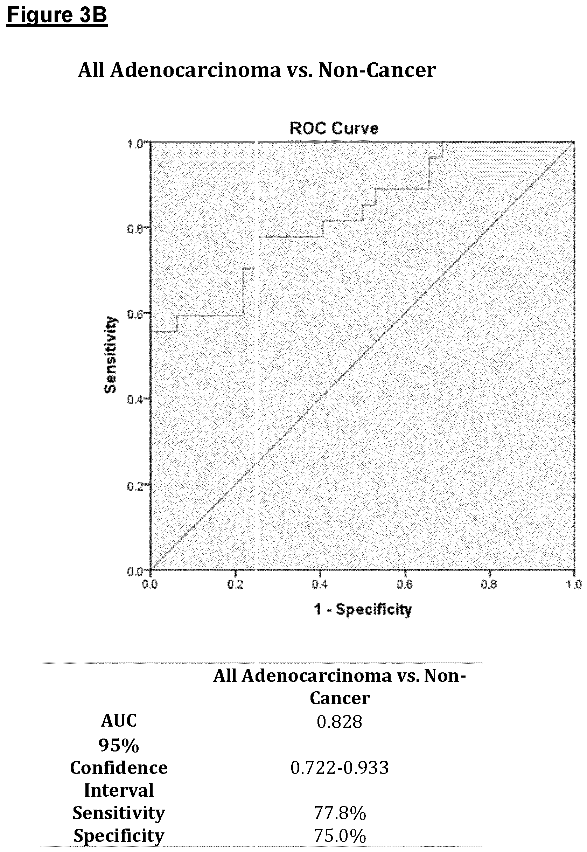

ROC plots (see Figure 2 and 3) were constructed for both cohorts using only VOCs that were found to be significantly altered in breath from cancer patients in the development cohort.

For the model Development study, ROC plots produced an AUC of 0.892 (95% CI, 0.806-0.978) for distinguishing Cancer vs. Non-Cancer, sensitivity 84% and specificity of 88.4%. The AUC produced from the Adenocarcinoma vs. Non-Cancer ROC was 0.943 (95% CI, 0.886-0.999) with a sensitivity of 88.2% and specificity of 90.7%. For the model Validation study, the AUC for distinguishing Cancer from Non-Cancer was 0.768 (95% CI, 0.65-0.885), producing a sensitivity of 78.8% and specificity of 75.0%. The AUC for distinguishing Adenocarcinoma from Non-Cancer was 0.851 (95% CI, 0.753-0.948) with a sensitivity of 85.2% and specificity of 70.0%.

### Discussion

Gas chromatography mass spectrometric quantification of VOCs in the exhaled breath has identified a total of 13 compounds that were significantly altered with the presence of pancreatic cancer. The significant VOCs were from three main chemical groups, namely aldehydes, fatty acids and alcohols. All ROC models showed good discrimination with AUCs over 0.7. Discrimination was also stronger in the models distinguishing adenocarcinoma from non-cancer. These results provide the foundation for a larger multi-centre study that could further establish the potential of breath VOC testing as a diagnostic tool for pancreatic cancer.

The chemical group with the largest number of significantly dysregulated breath VOCs in pancreatic cancer was the aldehyde group. Currently carbohydrate antigen 19-9 (CA19-9) is the most commonly used tumor marker for pancreatic cancer. However, it is often non-specific, being elevated in a number of both benign and malignant conditions including pancreatitis, cirrhosis, acute cholangitis and colorectal cancer [3]. It is also not expressed in 5-10% of the Caucasian population due to a Lewis a⁻/b⁻genotype [24]. Overall, only 65% of patients with surgically resectable pancreatic cancer will have elevated CA₁₉₋₉ [3]. Considering the discovery and early validation phase of this study, it is not advisable to make firm comparisons between breath VOC and CA19-9 testing.

The strength of the study lies in its novelty and design. The study provides the potential for non-invasive breath test to diagnose pancreatic cancer, a disease of unmet need that presents at a late stage with poor long-term survival. The advantages of design of the study include the inclusion of a positive control group, a reference test for each patient and an independent cohort of patients to validate volatile biomarkers employing a different breath collection method. The method adopted in the validation study lends itself towards multi-center clinical investigations, as ReCIVA provides a reproducible breath collection method while thermal desorption tubes offer a robust transport system that keeps volatile compounds stable for approximately 4 weeks.

The performance of the test should be examined in early pancreatic cancer as an ultimate goal for the breath test that could change the pattern of cancer stage at presentation and influence disease survival. The current study included patients with locally advanced and metastatic disease as this group represents the majority of patients with pancreatic cancer in clinical practice and should not be missed by the diagnostic model.

Breath VOC sampling is a completely non-invasive test with a very high acceptability by patients and clinicians as observed in the current study and others performed by our team [11,17,18,19]. The inventors envisage using exhaled breath testing as a triage investigation to establish the risk of pancreatic cancer in patients presenting with non-specific symptoms to guide referral for CT imaging. Another test location is screening for high-risk groups such as hereditary pancreatitis, familial pancreatic cancer, recent onset diabetes and intraductal papillary mucinous neoplasms. The final location of breath test in patient care pathway will depend on test sensitivity and specificity in large multicentre clinical trials and its performance in early pancreatic cancer stage and high-risk groups.

### Conclusions

Breath volatiles have the clear potential to distinguish pancreatic cancer from non-cancer patients.

### References

1. Siegel LR, Miller DK, Jemal A. Cancer Statistics 2017. CA Cancer J Clin 2017; 67: 7-30.
2. Li D, Xie K, Wolff R, et al. Pancreatic cancer. Lancet 2004; 363: 1049-57.
3. Goggins M. Molecular markers of early pancreatic cancer. J Clin Oncol 2005; 23: 4524 - 31.
4. Stapley S, Peters TJ, Neal RD, et al. The risk of pancreatic cancer in symptomatic patients in primary care: a large case-control study using electronic records. Br J Cancer 2012; 106: 1940 - 4.
5. PCUK. Study for survival. Secondary study for survival 2011. http: //www.pancreaticcancer.org.uk/media/100292/report .final
6. http://www.nice.org.uk/guidance/ng12/chapter/1-Recommendations-organised-by-site-of-cancer#upper-gastrointestinal-tract-cancers
7. Jenkinson C, Earl K, Ghaneh P, et al. Biomarkers for early diagnosis of pancreatic cancer. Expert Rev Gastroenterol Hepatol 2015; 9: 305-15.
8. Lennon AM, Wolfgang CL, Canto MI, et al. The early detection of pancreatic cancer: what will it take to diagnose and treat curable pancreatic neoplasia? Cancer Res 2014; 74: 3381-9.
9. Phillips M, Cataneo RN, Ditkoff BA, et al. Prediction of breast cancer using volatile biomarkers in the breath. Breast Cancer Res Treat 2006; 99: 19-21.
10. Kumar S, Huang J, Abbassi-Ghadi N, et al. Selected ion flow tube mass spectrometry analysis of exhaled breath for volatile organic compound profiling of esophago-gastric cancer. Anal Chem 2013; 85: 6121-8.
11. Kumar S, Huang J, Abbassi-Ghadi N, et al. Mass spectrometric analysis of exhaled breath for the identification of volatile organic compound biomarkers in esophageal and gastric adenocarcinoma. Ann Surg 2015; 262: 981-90.
12. Markar SR, Wiggins T, Kumar S, et al. Exhaled breath analysis for the diagnosis and assessment of endoluminal gastrointestinal diseases. J Clin Gastroenterol 2015; 49: 1-8.
13. Altomare DF, Di Lena M, Porcelli F, et al. Exhaled volatile organic compounds identify patients with colorectal cancer. Br J Surg 2013; 100: 144-50.
14. Phillips M, Gleeson K, Hughes JM, et al. Volatile organic compounds in breath as markers of lung cancer: a cross-sectional study. Lancet 1999; 353: 1930-3.
15. Markar SR, Lagergren J, Hanna GB. Research protocol for a diagnostic study of non-invasive exhaled breath analysis for the prediction of oesophago-gastric cancer. BMJ Open 2016; 6: e009139.
16. Markar SR, Chin ST, Romano A, et al. Breath volatile organic compound profiling of colorectal cancer using selected ion flow-tube mass spectrometry. Under review with Gastroenterology.
17. Boshier PR, Priest OH, Hanna GB, et al. Influence of respiratory variables on the on-line detection of exhaled trace gases by PTR-MS]. Thorax 2011; 66: 919-20.
18. Boshier PR, Cushnir JR, Priest OH, et al. Variation in the levels of volatile trace gases within three hospital environments: implications for clinical breath testing. J Breath Res 2010; 4: 031001.
19. Markar SR. Non-invasive volatile organic compound analysis from Exhaled Breath for the prediction of oesophago-gastric cancer. PhD thesis, Imperial College London 2017.
20. Poli D, Goldoni M, Corradi M, et al. Determination of aldehydes in exhaled breath of patients with lung cancer by means of on-fiber-derivatisation SPME-GC/MS. J Chromatogr B Anal Technol Biomed Life Sci 2010; 878: 2643-51.
21. Mochalski P, Sponring A, King J, et al. Release and uptake of volatile organic compounds by human hepatocellular carcinoma cells (HepG2) in vitro. Cancer Cell Int 2013; 13: 72.
22. Ma I, Allan AL. The role of human aldehyde dehydrogenase in normal and cancer stem cells. Stem Cell Rev Reports. 2011; 7: 292-306.
23. Deng S, Yang X, Lassus H, et al. Distinct expression levels and patterns of stem cell marker, aldehyde dehydrogenase isoform 1 (ALDH1), in human epithelial cancers. PLos One 2010; 5: e10277.
24. Rosen A Von, Linder S, Harmenberg U, et al. Serum CA 19-9 and CA 50 in relation to lewis blood cell status in patients with malignant and benign pancreatic disease. Pancreas 1993; 8: 160-5.

### Example 2 - Colorectal cancer

The following example is not part of the invention, and is present for illustration purposes only.

When colorectal cancer (CRCa) is diagnosed at its earliest stage, more than 9 in 10 people with CRCa will survive their disease for five years or more, compared with less than 1 in 10 when diagnosed at the latest disease stage [1]. However, the utilization of bowel symptoms as the primary diagnostic basis for CRCa has been shown to have a very poor positive predictive value [2]. This study identified and validated a unique breath volatile organic compound profile associated with the presence of colorectal cancer, suggesting the potential of breath analysis for inclusion in the colorectal cancer diagnostic pathway.

The objectives of this study were to: (i) identify changes in VOC concentration in exhaled breath of patients with CRCa compared to those with other colorectal diseases or normal lower gastrointestinal tract (control) from a prospective cohort study; (ii) validate these findings in a further prospectively collected cohort of patients with colorectal symptoms or disease; and (iii) identify changes in VOCs from exhaled breath observed with the presence of recurrence following CRCa surgical resection.

### Materials and Methods

### Sample Size:

Study (i) - Profiling diagnostic investigation in 150 patients (50 colorectal cancer; 50 positive controls including inflammatory bowel disease, polyps and diverticular disease and 50 negative controls with a normal lower gastrointestinal (LGI) tract endoscopy). Study (ii) - Independent diagnostic validation in 79 patients; 25 with CRCa and 54 control patients, which was a mix of positive and negative control patients.

Study (iii) - Clinical validation with tumour recurrence in 40 patients; 19 postoperative patients with no evidence of recurrence and 21 patients with documented hepatic or peritoneal recurrence. All patients were at least 6 months following initial colorectal surgery.

*Site:* St Mary's Hospital.

*Ethical approval:* NHS Health Research Authority (NRES Committee London - Camden and Islington) approval gained on 16^{th} July 2014 (REC reference 14/LO/1136). Trials registration number: UKCRN18063

*Inclusion criteria:* Patients attending St Mary's Hospital for lower gastrointestinal endoscopy or surgery for colorectal cancer or investigation of LGI symptoms (studies (i) and (ii)). Patients attending oncological clinic for follow-up were recruited for study (iii).

*Exclusion criteria:* Patients with a documented active infection or liver diseases.

*Breath sampling methodology:* All patients were asked to sign a consent form to be included in this study. The inventors followed the sampling protocol used in previous clinical studies [9] that was informed by their investigations on the influence of breath manoeuvres and hospital environment on VOC measurement [14,15]. Patients were fasted for a minimum of four hours prior to their breath sample collection. Patients were rested in the same area for at least 20minutes prior to breath sampling and all breath samples were retrieved prior to endoscopy or surgery. Patients were asked to perform a single deep nasal inhalation followed by complete exhalation via their mouth into secure double thickness (2 x25µm) Nalophan (Kalle UK Ltd., Witham, UK) bags via a 1mL Luer lok syringe (Terumo Europe, Leuven, Belgium).

*Sample analysis:* For each VOC measurement, the syringe plunger was removed from the 1ml Luer lok syringe and the Nalophan bag was directly connected via the syringe barrel to the sample inlet arm of the SIFT-MS instrument. For the multi-ion monitoring mode, selective VOCs from breath were analysed for a total of 60s and measured concentrations were averaged over this time for each VOC. As this was a single centre study, each breath sample was analysed within one hour of the sample being taken from the patient and therefore Nalophan breath bags were used as in previous research conducted by our group [9]. The methodological studies demonstrated the stability of trace VOCs up to two hours from the time of patient sampling [16].

*Selected Ion Flow-tube Mass Spectrometry (SIFT-MS):* SIFT-MS permits online, real-time VOC quantification [11,12]. The principle of SIFT-MS is based upon direct mass spectrometric analysis by chemical ionisation of the VOCs in air or vapour samples. Selected precursor ions (H₃O⁺, NO⁺ and O₂⁺) are injected into the helium carrier gas, and ionise the VOCs within the breath samples, with the generation of characteristic product ions, which are detected by the quadrupole mass spectrometer downstream. By measuring the count rate of both precursor ions and the characteristic product ions at the downstream detection system, a real-time quantification is achieved, realising the absolute concentration of trace and volatile compounds at the parts-per-billion by volume or parts-per-million by volume. SIFT-MS has been utilised in the study of VOCs in breath and urine from patients with conditions including cystic fibrosis and bladder cancer [17,18]. The SIFT-MS technique allows real-time detection and quantification of VOCs within biological samples such as exhaled breath without sample preparation [19]. The inventors have previously confirmed the reproducibility of VOCs measurements using SIFT-MS [20].

*Clinical data:* A detailed medical proforma was completed by the consenting medical practitioner or research fellow using information provided by the patient as well as clinical investigations. These data included patient demographics, tumour characteristics, comorbidities, medications and lifestyle measures. Diagnostic endoscopy and/or operative findings were recorded for each patient.

*Criterion standard for comparison:* Endoscopy findings confirmed with histological examination were used as the gold standard for patient classification into the CRCa or control cohorts. For post-operative patients the latest CT scan performed as part of routine follow-up was used to identify those patients with post-operative recurrence.

*Statistical analysis:* SIFT-MS output data provides measured concentration of VOCs from exhaled breath. These concentrations were then compared using univariate statistics, Kruskal-Wallis test, across the study groups. VOCs that were significant in univariate statistics were then taken forward into a multivariable logistic regression model with the dependent variable being the presence of CRCa. To construct the Receiver Operating Characteristic (ROC) curves, cancer status was used as the dependent variable and the sum concentrations of significant VOCs from the multivariable logistic regression model were used as the independent variables. P value of <0.05 was used to assign statistical significance. All statistical analysis was performed using the statistical software SPSS (version 22).

*Cross-platform GC-MS validation:* In order to confirm the identify VOCs obtained in the exhaled breath using SIFT-MS, the inventors conducted cross platform validation with GC-MS being the standard separation technique. Exhaled breath was collected using the same methodology from 20 patients. The VOC content from each Nalophan breath bag was transferred using a SKC 210-1002 Series air-sampling pocket pump (PA, USA) at 50 mL/min onto an inert coated stainless steel Tenax/Carbograph-5TD sorbent tubes (Markes International Ltd, Llantrisant, UK) prior to GC-MS analysis. An Agilent 7890B GC with 5977A MSD (Agilent Technologies, Cheshire, UK), coupling to a Markes TD-100 thermal desorption unit (TDU) was used. A two-stage thermal desorption program was used at 50 mL/min constant Helium flow rate. In the primary desorption stage, the TD tube sample was dry-purged for 3 min before heated to 280 °C for 10 min. During secondary desorption stage, VOC from the cold trap (U-T12ME-2S) was rapidly desorbed from 10 °C to 290 °C at 99 °C/min heating rate and held for 4 min to completely transfer the VOCs onto GC. Flow path from TDU to GC was heated constantly at 140 °C.

VOCs separation was performed on a ZB-624 capillary column (60 m × 0.25 mm ID × 1.40 µm *d_{f}*; Phenomenex Inc, Torrance, USA) programmed at 1.0 mL/min Helium carrier. Oven temperature profile was set at 40 °C initially for 4 min, ramp to 100 °C (5 °C/min with 1 min hold), ramp to 110 °C (5 °C/min with 1 min hold), ramp to 200 °C (5 °C/min with 1 min hold), final ramp to 240 °C at 10 °C/min with 4 min hold. The MS transfer line was maintained at 240 °C whilst the EI source was set at 70 eV and 230 °C. MS analyser was set to acquire over the range of 20-250 m/z with data acquisition approximated to 6 scan/sec. The GC-MS data was processed using MassHunter software version B.07 SP1 (Agilent Technologies) while MS data of the separated VOC component is compared with NIST Mass Spectral Library (National Institute of Standards and Technology version 2.0) for identification.

### Results

### Study (i) - profiling diagnostic investigation

150 patients (50 patients with colorectal cancer, 50 patients with other conditions of the lower gastrointestinal tract (positive controls), and 50 patients with a normal lower gastrointestinal tract (negative controls)). Of the positive control patients, 15 (30 %) had inflammatory bowel disease, 21 (42%) had polyps within the lower gastrointestinal tract and 14 (28%) had diverticular disease. All patients with diverticular disease and inflammatory bowel disease did not have active diverticulitis or colitis at the time of breath sampling.

Comparative analysis of patient medical comorbidities revealed no significant differences between the groups with the exception of an increase in the proportion of patients aged 70 years or older in the CRCa group (see Table 1a).

**Table 1a: Comparison of patient medical comorbidities**

| **Comorbidity** | **All patients (n=150) (%)** | **Negative control (n=50)(%)** | **Positive control (n=50)(%)** | **CRCa (n=50) (%)** | **P value** |
|---|---|---|---|---|---|
| Age ≥70* | 36 (26) | 5 (10) | 9 (20) | 22 (28) | <0.001 |
| Male gender | 79 (53) | 22 (44) | 25 (54) | 30 (60) | 0.27 |
| Ethnicity: | | | | | |
| White | 97 (65) | 29 (58) | 35 (70) | 33 (66) | 0.23 |
| Black | 9 (6) | 2 (4) | 2 (4) | 5 (10) | |
| Asian | 20 (13) | 7 (14) | 8 (16) | 5 (10) | |
| Arab | 11 (7) | 7 (14) | 2 (4) | 2 (4) | |
| Previous* cancer | 17 (12) | 5 (10) | 10 (20) | 2 (4) | 0.07 |
| Diabetes | 15 (10) | 5 (10) | 3 (6) | 7 (14) | 0.33 |
| Renal disease* | 5 (3) | 2 (4) | 1 (2) | 2(4) | 0.81 |
| COPD* | 14 (10) | 4 (8) | 5 (10) | 5 (11) | 0.89 |
| IHD* | 13 (9) | 4 (8) | 2 (4) | 7 (15) | 0.17 |
| Liver disease* | 5 (4) | 1 (2) | 4 (8) | 0 (0) | 0.07 |
| Hypertension* | 37 (26) | 9 (18) | 15 (32) | 13 (28) | 0.29 |
| Asthma* | 14 (10) | 6 (12) | 5 (10) | 3 (7) | 0.63 |
| Previous surgery* | 57 (41) | 18 (37) | 23 (48) | 16 (37) | 0.46 |
| Smoking history*: | | | | | |
| Ex | 25 (17) | 7 (14) | 8 (16) | 10 (20) | 0.57 |
| Current | 25 (17) | 11 (22) | 9 (18) | 5 (10) | |
| Alcohol history*: | | | | | |
| Ex | 17 (12) | 3 (6) | 7 (15) | 7 (15) | 0.63 |
| Current | 71 (50) | 25 (51) | 23 (48) | 23 (50) | |
| Use of bowel preparation | 112 (75) | 46 (92) | 36 (72) | 30 (60) | 0.01 |

| | | | | | |
|---|---|---|---|---|---|
| *7 patients with missing data | | | | | |

There was also an increased proportion of patients receiving bowel preparation in the control groups (Table 1a). Comparative analysis of presenting symptoms that stimulated referral for lower gastrointestinal endoscopy showed no significant differences between the groups with the exception of an increased proportion of patients with anaemia in the CRCa group (see Table 1b).

**Table 1b: Comparison of presenting symptoms**

| **Symptom** | **All patients (n=150) (%)** | **Negative control (n=50) (%)** | **Positive control (n=50) (%)** | **CRCa (n=50) (%)** | **P value** |
|---|---|---|---|---|---|
| PR Bleeding | 76 (51) | 30 (60) | 23 (46) | 23 (46) | 0.27 |
| Change in bowel habit | 33 (22) | 12 (24) | 12 (24) | 9 (18) | 0.71 |
| Tenesmus* | 3 (2) | 1 (2) | 1 (2) | 1 (2) | 0.99 |
| Mucus* | 2 (1) | 0 (0) | 2 (4) | 0 (0) | 0.14 |
| Abdo pain* | 21 (15) | 7 (14) | 8 (17) | 6 (13) | 0.88 |
| Bloating* | 8 (6) | 3 (6) | 3 (6) | 2 (4) | 0.91 |
| Appetite* | 2 (1) | 1 (2) | 0 (0) | 1 (2) | 0.60 |
| Weight loss* | 23 (16) | 6 (12) | 6 (13) | 11 (24) | 0.21 |
| Anaemia* | 15 (10) | 2 (4) | 4 (8) | 9 (20) | 0.04 |

| | | | | | |
|---|---|---|---|---|---|
| *7 patients with missing data | | | | | |

Comparative analysis of utilisation of medication between the groups, showed an increase use of proton pump inhibitor and clopidogrel within the CRCa group (see Table 1c).

**Table 1c: Comparison of utilisation of medication**

| **Medication** | **All patients (n=150) (%)** | **Negative control (n=50) (%)** | **Positive control (n=50) (%)** | **CRCa (n=50) (%)** | **P value** |
|---|---|---|---|---|---|
| PPI* | 34 (24) | 12 (24) | 5 (10) | 17 (37) | 0.01 |
| Statin | 25 (17) | 4 (8) | 10 (20) | 11 (22) | 0.13 |
| Beta-blocker* | 16 (11) | 4 (8) | 4 (8) | 8 (16) | 0.33 |
| ACE inhibitor* | 14 (10) | 3 (6) | 5 (10) | 6 (13) | 0.52 |
| Amlodipine* | 14 (10) | 3 (6) | 5 (10) | 6 (13) | 0.52 |
| Aspirin* | 13 (9) | 5 (10) | 2 (4) | 6 (13) | 0.31 |
| Clopidogrel* | 3 (2) | 0 (0) | 0 (0) | 3 (7) | 0.04 |
| Metformin* | 14 (10) | 4 (8) | 4 (8) | 6 (13) | 0.67 |
| Furosemide* | 4 (3) | 0 (0) | 2 (4) | 2 (4) | 0.34 |

| | | | | | |
|---|---|---|---|---|---|
| *7 patients with missing data | | | | | |

Of the 50 patients with CRCa, 52% were tumours of the rectum, 66% moderately differentiated tumours, 48% T3 and 52 % No tumours, and 34% of patients received neoadjuvant therapy preoperatively (see Table 2).

**Table 2: Tumour characteristics of colorectal cancer patients included**

| **Demographic** | **Patient Number (%)** |
|---|---|
| Tumour location: | |
| Rectum | 26 (52) |
| Descending/Sigmoid colon | 9 (18) |
| Caecum/Ascending/Transverse colon | 13 (26) |
| Synchronous (Left and Right) | 2 (4) |
| Tumour differentiation: | |
| Good | 1 (2) |
| Moderate | 33 (66) |
| Poor | 8 (16) |
| R1/2 margin | 4 (8) |
| Pathological stage | |
| T0 | 2 (4) |
| T1 | 3 (6) |
| T2 | 6 (12) |
| T3 | 24 (48) |
| T4 | 8 (16) |
| Pathological stage | |
| N0 | 26 (52) |
| N1 | 12 (24) |
| N2 | 5 (10) |
| Dukes | |
| A | 9 (18) |
| B | 15 (30) |
| C | 18 (36) |
| D | 1 (2) |
| Lymph nodes harvested | 21 (13 - 30) |
| Lymph nodes positive | 0 (0 - 1) |
| Neoadjuvant chemo/CRx | 17 (34) |

Seven compounds were found to be significantly different between cancer and control groups, of which only propanal (NO+) was significantly associated with colorectal cancer in multivariate analysis (see Table 3).

**Table 3: SIFT-MS Exhaled Breath analysis of Colorectal patients**

| **VOC** | **All patients (n=150) (%)** | **Negative control (n=50) (%)** | **Positive control (n=50) (%)** | **CRCa (n=50) (%)** | **P value** |
|---|---|---|---|---|---|
| Ammonia (H30) | 42195 (31605 - 51431) | 41374 (28980 - 50173) | 37392 (24479 - 48556) | 46588 (36371 - 61320) | 0.01 |
| Ethanol (H30) | 145 (84 - 257) | 165 (111 - 297) | 71 (41 - 151) | 182 (121 - 376) | <0.001 |
| Acrolein (H30) | 9 (5 - 17) | 7 (4 - 12) | 7 (2 - 13) | 13 (7 - 20) | 0.02 |
| Propanol (H30) | 48 (32 - 86) | 42 (27 - 69) | 50 (38 - 96) | 56 (38 - 102) | 0.02 |
| Butanol (H30) | 20 (11 - 33) | 16 (10 - 24) | 20 (12 - 30) | 26 (38 - 103) | 0.04 |
| Propanal (N0)** | 25 (1.5 - 39) | 18 (11 - 21) | 21 (14 - 26) | 31 (25 - 45) | <0.001 |
| Carbon disulphide (02) | 160 (124 - 256) | 150 (123 - 215) | 152 (114 - 248) | 192 (144 - 273) | 0.02 |

| | | | | | |
|---|---|---|---|---|---|
| ** only VOC significant in multivariate analysis | | | | | |

Previous investigation has demonstrated reliable quantification of propanal aldehyde by SIFT-MS due to specific hydride transfer reaction occurs between saturated aldehyde and NO+ reagent [18]. In parallel to SIFT-MS analysis, cross-platform analysis by GC-MS unravels that the propanal peak was distinctly observed in the gas chromatogram at retention time (RT) of 8.92 min among the cancer breath samples. The selected ZB-624 capillary column has provided sufficient resolution efficiency to separate propanal from the abundant acetone peak (RT of 9.11 min) although they both possesses the common fragment ion of 58 m/z. The inventors have shown high similarity of the deconvoluted mass spectral details of propanal peak (character ions of 29 m/z, 39 m/z and 58 m/z) with the NIST library matching.

Table 4 shows a range of VOCs which were tested as biomarkers, and again propanal was shown to be significantly associated with colorectal cancer in multivariate analysis.

**Table 4: SIFT-MS Exhaled Breath analysis of Colorectal patients**

| **VOC** | **Formulae** | **Precursor ion** | **Mass/charge product ion(s)** |
|---|---|---|---|
| Acetone | C₃H₆O | NO+ | 88 |
| Acetic acid | C₂H₄O² | NO+ | 90, 108 |
| Isoprene | C₅H₈ | NO+ | 68 |
| **Propanal*** | C₂H₅CHO | NO+ | 57 |
| Butanal | C₃H₇CHO | NO+ | 71 |
| Pentanal | C₄H₉CHO | NO+ | 85 |
| Hexanal | C₅H₁₁CHO | NO+ | 99 |
| Heptanal | C₆H₁₃CHO | NO+ | 113 |
| Octanal | C₇H₁₅CHO | NO+ | 127 |
| Nonanal | C₈H₁₇CHO | NO+ | 141 |
| Decanal | C₉H₁₉CHO | NO+ | 155 |
| Methanol | CH₄0 | H30+ | 33, 51 |
| Propanol* | C₃H₈0 | H30+ | 43 |
| Butanol | C₄H₁₀O | H30+ | 57 |
| Pentanol | C₅H₁₂0 | H30+ | 71 |
| Pentanoic acid | C₅H₁₀O₂ | H30+ | 103 |
| Hexanoic acid | C₆H₁₂O₂ | H30+ | 117, 135 |
| Hydrogren sulphide | H₂S | H30+ | 35 |
| Hydrogren cyanide | HCN | H30+ | 28 |
| Acetaldehyde | C₂H₄O | H30+ | 45, 81 |
| Formaldehyde | H₂CO | H30+ | 31 |
| Phenol | C₆H₆O | NO+ | 94, 112 |
| Methyl phenol | C₇H₈O | O2+ | 108, 126 |
| Ethyl phenol | C₈H₁₀O | NO+ | 122, 140 |
| Ammonia | NH3 | O2+ | 17, 35 |

| | | | |
|---|---|---|---|
| *VOC significantly associated with colorectal cancer in multivariate analysis. | | | |

Propanal (NO+) performed well as a single breath biomarker for colorectal cancer when compared to negative controls (area under the ROC curve = 0.90 ± 0.03 (see Figure 1a)) and positive controls (area under the ROC curve = 0.83 ± 0.04 (see Figure 1b)). In distinguishing CRCa from negative controls propanal as a single breath biomarker based on a threshold of 28ppbv, had a sensitivity of 96% and specificity of 76%. Propanal at a threshold of 28ppbv was also able to distinguish CRCa from positive control patients with a sensitivity of 90% and specificity of 66%.

To ensure the elevated propanal concentration seen in the CRCa was not the result of a confounding factor that differed between the groups, multivariable linear regression analysis was performed and demonstrated that the presence of CRCa was the sole factor significantly associated with elevated levels of propanal.

### Study (ii) - independent diagnostic validation

In total 79 patients were studied, 25 with CRCa and 54 control patients. Of the 54 control patients, 31 (57.4%) had a normal lower gastrointestinal tract on endoscopy, 12 (22%) had polyps, 7 (13%) had diverticular disease and 4 (7%) had inflammatory bowel disease. Comparative analysis of patient medical comorbidities, use of medication and presenting symptoms revealed no significant differences between the groups with the exception of an increase in the proportion of patients aged 70 years or older and increased presentation with anaemia in the colorectal cancer group.

Propanal was significantly elevated in the CRCa group when compared with the control group (median 30 vs. 19 ppbv; P<0.01). Propanal (NO+) performed well as a single breath biomarker for CRCa when compared with control patients with an area under the ROC curve of 0.79 ± 0.06 (Figure 2). Again, using a threshold of 28ppbv this gave propanal a sensitivity of 83.3% and specificity of 84.7% for the diagnosis of CRCa.

### Study (iii) - clinical validation with tumor recurrence

In total 40 patients were studied, 19 postoperative patients with no evidence of recurrence and 21 patients with documented recurrence. Of the patients with recurrence, 5 patients had isolated hepatic recurrence, and 16 patients had either peritoneal recurrence alone or hepatic and peritoneal recurrence simultaneously. Comparative analysis of patient medical comorbidities, and use of medication revealed no significant differences between the groups.

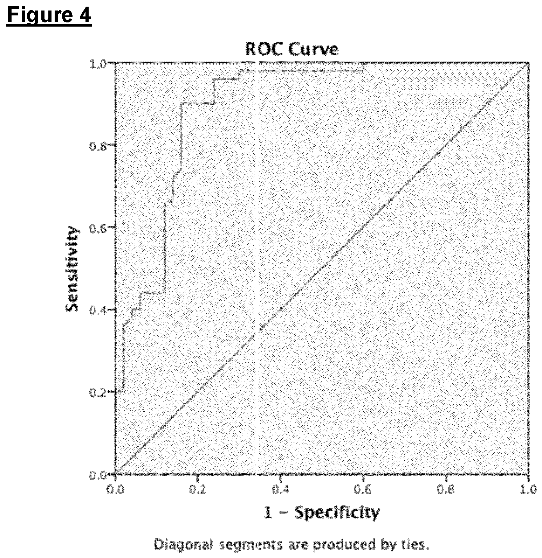

Propanal concentration was significantly elevated in patients with CRCa recurrence compared to the non-recurrence group (median 38 vs. 19ppbv; P<0.01). Propanal (NO+) was employed as a single breath biomarker in identifying postoperative patients with CRCa recurrence with an area under the ROC curve of 0.81 ± 0.07 (Figure 3). Using a threshold of 28ppbv, this gave propanal a sensitivity of 71.4% and a specificity of 90.9% in identifying patients with recurrence following surgery. Figure 4 shows the changes in propanal concentration across the four disease states.

Across all three studies all patients invited to participate in the study, accepted the invitation and took part in the study, giving a patient acceptability rate of 100%.

### Discussion

The results of this study suggest that propanal has the potential to be a single breath biomarker for the diagnosis of CRCa. Propanal appeared to distinguish with a good diagnostic accuracy patients with CRCa from patients with other colorectal diseases and subjects with an endoscopically normal lower gastrointestinal tract. Those findings have been validated in an independent diagnostic study and in patients with colorectal cancer recurrence. The elevation in propanal concentration was not influenced by patient factors as shown in multivariate analysis. Propanal was not a component of previously generated and validated VOC breath model for oesophago-gastric cancer [9] suggesting that cancers of upper and lower gastrointestinal tract have distinct nonoverlapping breath profiles that may permit cancer-specific analysis in future clinical practice.

A previous study by Altomare et al using GC-MS identified 15 VOCs from exhaled breath that significantly differed between CRCa and control patients [10]. Of these nonanal and decanal were the only aldehydes detected. Altomare et al, did attempt to validate their findings in a prospective cohort of patients, however the initial model that was developed in 78 patients was validated in only 25 patients. The methodology employed by Altomare et al used underivitised analysis of aldehydes on GC-MS, which permits measurement of long-chain aldehydes such as nonanal and decanal, but not shorter aldehydes such as propanal, which are lost as they are more volatile. This may be a major factor in the difference in results observed between our investigation and the Altomare study [10]. Furthermore a recent study by Amal et al, similarly using GC-MS did not identify aldehydes in exhaled breath that differed between CRCa and control groups [21]. The different mass spectrometry techniques may account for the differences between studies. A combination of short and long chain aldehydes as the basis for the diagnostic model in CRCa need further investigation.

Propanal (CH₃CH₂CHO) is a saturated 3-carbon aldehyde and is a structural isomer of acetone. Cross platform validation using gas chromatography mass-spectrometry confirmed propanal identity. The most plausible explanation for the elevation in propanal concentration is the derangement of aldehyde metabolism in colorectal cancer [22]. Specifically aldehyde dehydrogenase-1 expression has been demonstrated to be dysregulated in colorectal cancer, and independently affect long-term survival, along with response to neoadjuvant chemotherapy [23,24]. Another contributing factor to the elevation of propanal is the changes in the gut microbiota and the complex interplay between the microbiome and the adaptive immune response in cancer development [25,26].

As an initial investigation towards the development of a breath test for colorectal cancer, the advantages of these studies include: (i) the discovery of a single volatile biomarker, (ii) setting up a test threshold for diagnosis using quantitative mass spectrometry technique that does not require sample preparation; (iii) the conduction of independent validation in two prospective cohorts of patients with CRCa and with tumour recurrence after resection and (iv) all patients had lower gastrointestinal endoscopy as the criterion standard for comparison. Using a threshold of 28ppbv, it was possible to distinguish cancer from control groups with a diagnosis accuracy ranging from 79% to 90%, and is comparable with faecal occult blood and faecal immunochemical tests that are currently in clinical practice [3-6]. Previous breath studies have largely relied upon the combination of changes in several VOCs to generate a risk prediction model [9,10,21]. Only few studies have demonstrated the association of a single VOC biomarker with a disease state; hydrogen cyanide with pseudomonas aeruginosa infection, and pentane with inflammatory bowel disease [27,28]. On the other hand, the inventors' investigations have the limitations of a single centre study in both clinical and analytical aspects. A larger multicentre study that is powered to provide a definitive answer for test accuracy is currently being planned. The performance of breath test requires investigations in early stages of CRCa. Although the inventors have shown repeatability of experimental measurements over a short time period [20], the reproducibility of the results using mass-spectrometry instruments in different laboratories or with the same instruments in a central laboratory over a long period of time should be carefully examined before the clinical uptake of any proposed breath test. Furthermore, the mechanism of propanal production and aldehyde dysregulation remains an important area for future work that can improve the understanding of confounding factors and improve the control measures on administering the test.

The discovery described herein and validation experiments were conducted in symptomatic colorectal patients with colorectal cancer, benign disease or endoscopically normal colon. While planning a definitive diagnostic accuracy study, it is important to study the proposed location of an exhaled breath test for colorectal cancer. The inventors envisage the use of such test as a triage investigation to direct patients to have endoscopy. If the test has an acceptable sensitivity and specificity and patient acceptability, it may prove cost-effective and improve the diagnostic pathway in patients with colorectal symptoms; an impact that requires several studies to investigate, yet an important vision to outline at early stage.

### Conclusions

This study suggests the association of a single breath biomarker (propanal) with the primary presence and recurrence of CRCa. Further multi-centre validation studies are required to validate these findings.

### References

1. Torre LA, Bray F, Siegel RL, et al. Global cancer statistics, 2012. CA Cancer J Clin 2015; 65: 87-108.
**2.** Ewing M, Naredi P, Zhang C, et al. Identification of patients with non-metastatic colorectal cancer in primary care: a case-control study. Br J Gen Pract 2016; 66: e880-e886.
**3.** Lieberman DA, Weiss D; Veterans Affairs Cooperative Study Group 380. One-time screening for colorectal cancer with combined fecal occult-blood testing and examination of the distal colon. N Engl J Med 2001; 345: 555-560.
**4.** Imperiale TF, Ranshoff DF, Itzkowitz SH, et al. Fecal DNA versus fecal occult blood for colorectal-cancer screening in an average-risk population. N Engl J Med 2004; 351: 2704-2714.
**5.** Allison JE, Tekawa IS, Ransom LJ, et al. A comparison of fecal occult-blood tests for colorectal-cancer screening. N Engl J Med 1996; 334: 155-159.
**6.** Allison JE, Sakoda LC, Levin TR, et al. Screening for colorectal neoplasms with new fecal occult blood tests: update on performance characteristics. J Natl Cancer Inst. 2007; 99: 1462-1470.
**7.** Imperiale TF, Ransohoff DF, Itzkowitz SH, et al. Multitarget stool DNA testing for colorectal-cancer screening. N Engl J Med 2014; 370: 1287-1297.
**8.** Nakhleh MK, Amal H, Jeries R, et al. Diagnosis and classification of 17 diseases from 1404 subjects via pattern analysis of exhaled molecules. ACS Nano 2017; 11: 112-125.
**9.** Kumar S, Huang J, Abbassi-Ghadi N, et al. Mass spectrometric analysis of exhaled breath for the identification of volatile organic compound biomarkers in esophageal and gastric adenocarcinoma. Ann Surg 2015; 262; 981-990.
**10.** Altomare DF, Di Lena M, Porcelli F, et al. Exhaled volatile organic compounds identify patients with colorectal cancer. Br J Surg 2013; 100: 144-150
**11.** Spanel P, Smith D. Selected ion flow tube mass spectrometry for on-line trace gas analysis in biology and medicine. Eur J Mass Spectrom 2007; 13: 77-82
**12.** Spanel P, Smith D, Progress in SIFT-MS: breath analysis and other applications. Mass Spectrom Rev 2011; 30: 236-267
**13.** Altomare DF, Di Lena M, Porcelli F, et al. Effects of curative colorectal cancer surgery on exhaled volatile organic compounds and potential implications in clinical follow-up. Ann Surg 2015; 262: 862-866.
**14.** Boshier PR, Cushnir JR, Priest OH, et al. Variation in the levels of volatile trace gases within three hospital environments: implications for clinical breath testing. J Breath Res 2010; 4: 031001.
**15.** Boshier PR, Priest OH, Hanna GB, et al. Influence of respiratory variables on the on-line detection of exhaled trace gases by PTR-MS. Thorax 2011; 66: 919-920.
**16.** Markar SR. Non-invasive volatile organic compound analysis from Exhaled Breath for the prediction of oesophago-gastric cancer. PhD thesis, Imperial College London 2017.
**17.** Spanel P, Smith D, Holland TA, et al. Analysis of formaldehyde in the headspace of urine from bladder and prostate cancer patients using selected ion flow tube mass spectrometry. Rapid Commun Mass Spectrom. 1999; 13: 1354-1359
**18.** Smith D, Sovova K, Dryahina K, et al. Breath concentration of acetic acid vapour is elevated in patients with cystic fibrosis. J Breath Res 2016; 10: 021002.
**19.** Boshier PR, Cushnir JR, Mistry V, et al. On-line, real time monitoring of exhaled trace gases by SIFT-MS in the perioperative setting: a feasibility study. Analyst 2011; 136: 3233-3237.
**20.** Boshier PR, Marczin N, Hanna GB. Repeatability of the measurement of exhaled volatile metabolites using selected ion flow tube mass spectrometry. J Am Soc Mass Spectrom 2010; 21: 1070-1074.
**21.** Amal H, Leja M, Funka K, et al. Breath testing as potential colorectal cancer screening tool. Int J Cancer 2016; 138: 229-236.
**22.** Brown DG, Rao S, Weir TL, et al. Metabolomics and metabolic pathway networks from human colorectal cancers, adjacent mucosa, and stool. Cancer Metab 2016; 4: 11.
**23.** Li H, Jiang Y, Pei F, et al. Aldehyde dehydrogenase 1 and nodal as significant prognostic markers in colorectal cancer. Pathol Oncol 2016; 22: 121-7.
**24.** Deng Y, Zhou J, Fang L, et al. ALDH1 is an independent prognostic factor for patients with stages II-III rectal cancer after receiving radiochemotherapy. Br J Cancer 2014; 110: 430-4.
**25.** Vogtmass E, Hua X, Zeller G, et al. Colorectal cancer and human gut microbiome: reproducibility with whole-genome shotgun sequencing. PLos One 2016; 11: e0155362.
**26.** Lasry A, Zinger A, Ben-Neriah Y. Inflammatory networks underlying colorectal cancer. Nat Immunol 2016; 17: 230-40.
**27.** Dryahina K, Spanel P, Pospisilova V, et al. Quantification of pentane in exhaled breath, a potential biomarker of bowel disease, using selected ion flow tube mass spectrometry. Rapid Commun Mass Spectrom. 2013; 27: 198 -192
**28.** Smith D, Spanel P, Gilchrist FJ. Hydrogen cyanide, a volatile biomarker of pseudomonas aeruginosa infection. J Breath Res 2013; 7: 044001.

## Claims

1. A method for diagnosing a subject suffering from pancreatic cancer, or a pre-disposition thereto, or for providing a prognosis of the subject's condition, the method comprising analysing the concentration of a signature compound in a breath sample from a test subject and comparing this concentration with a reference for the concentration of the signature compound in an individual who does not suffer from pancreatic cancer, wherein a decrease in the concentration of the signature compound selected from a C₁-C₂₀ alkane in the breath sample from the test subject, compared to the reference, suggests that the subject is suffering from pancreatic cancer, or has a pre-disposition thereto, or provides a negative prognosis of the subject's condition.

2. A method for determining the efficacy of treating a subject suffering from pancreatic cancer with a therapeutic agent or a specialised diet, the method comprising analysing the concentration of a signature compound in a breath sample from a test subject and comparing this concentration with a reference for the concentration of the signature compound in an individual who does not suffer from pancreatic cancer, wherein an increase in the concentration of the signature compound selected from a C₁-C₂₀ alkane in the breath sample from the test subject, compared to the reference, suggests that the treatment regime with the therapeutic agent or the specialised diet is effective, or wherein a decrease in the concentration of the signature compound selected from a C₁-C₂₀ alkane in the breath sample from the test subject, compared to the reference, suggests that the treatment regime with the therapeutic agent or the specialised diet is ineffective.

3. A method according to either claim 1 or claim 2, wherein the signature compound is a C₅ alkane.

4. A method according to either claim 1 or claim 2, wherein the signature compound is a C₆ alkane.

5. A method according to either claim 1 or claim 2, wherein the signature compound is a C₁₄ alkane.

6. A method according to any one of claims 1, 3, 4 or 5, wherein (i) an increase in the concentration of an additional signature compound selected from a C₁-C₃ aldehyde, C₁-C₃ alcohol, and C₂-C₁₀ alkane wherein a first carbon atom of the C₂-C₁₀ alkane is substituted with a =O group and a second carbon atom is substituted with an -OH group, in the breath sample from the test subject, or (ii) a decrease in the concentration of an additional signature compound selected from a C₄-C₁₀ alcohol, C₁-C₆ carboxylic acid, and C₄-C₂₀ aldehyde, in the breath sample from the test subject, compared to the reference, suggests that the subject is suffering from pancreatic cancer, or has a pre-disposition thereto, or provides a negative prognosis of the subject's condition.

7. A method according to any one of claims 2 to 5, wherein (i) a decrease in the concentration of an additional signature compound selected from C₁-C₃ aldehyde, C₁-C₃ alcohol, and C₂-C₁₀ alkane wherein a first carbon atom of the C₂-C₁₀ alkane is substituted with a =O group and a second carbon atom is substituted with an -OH group, in the breath sample from the test subject, compared to the reference, or (ii) an increase in the concentration of an additional signature compound selected from a C₄-C₁₀ alcohol, C₁-C₆ carboxylic acid, and C₄-C₂₀ aldehyde, in the breath sample from the test subject, compared to the reference, suggests that the treatment regime with the therapeutic agent or the specialised diet is effective, or wherein (i) an increase in the concentration of an additional signature compound selected from a C₁-C₃ aldehyde, C₁-C₃ alcohol, and C₂-C₁₀ alkane wherein a first carbon atom of the C₂-C₁₀ alkane is substituted with a =O group and a second carbon atom is substituted with an -OH group, in the breath sample from the test subject, compared to the reference, or (ii) a decrease in the concentration of an additional signature compound selected from a C₄-C₁₀ alcohol, C₁-C₆ carboxylic acid, and C₄-C₂₀ aldehyde, in the breath sample from the test subject, compared to the reference, suggests that the treatment regime with the therapeutic agent or the specialised diet is ineffective.

8. A method according to either claim 6 or claim 7, wherein when the additional signature compound is a C₁-C₃ aldehyde, the compound is a C₁ aldehyde, and/or
wherein when the additional signature compound is a C₁-C₃ alcohol, the compound is a C₁ alcohol or a C₃ alcohol.

9. A method according to any one of claims 5 to 8, wherein when the additional signature compound is a C₂-C₁₀ alkane wherein a first carbon atom is substituted with the =O group and a second carbon atom is substituted with an -OH group, the carbon atom substituted with the =O group is not a terminal carbon atom, and/or
wherein when the additional signature compound is a C₂-C₁₀ alkane wherein a first carbon atom is substituted with the =O group and a second carbon atom is substituted with an -OH group, the compound is a C₃-C₆ alkane or a C₄ alkane wherein a first carbon atom is substituted with the =O group and a second carbon atom is substituted with an -OH group.

10. A method according to any one of claims 5 to 9, wherein:
(i) when the additional signature compound is a C₄-C₁₀ alcohol, the compound is a C₄-C₇alcohol or a C₄ alcohol;
(ii) when the additional signature compound is a C₁-C₆ carboxylic acid, the compound is a C₂-C₄ carboxylic acid or a C₃ carboxylic acid;
(iii) when the additional signature compound is a C₄-C₂₀ aldehyde, the compound is a C₅-C₁₅ aldehyde or C₇-C₁₃ aldehyde; and/or
(ii) when the additional signature compound is a C₄-C₂₀ aldehyde, the compound is a C₈ aldehyde, or a C₉ aldehyde, or a C₁₀ aldehyde, or a C₁₁ aldehyde.

11. Use of an apparatus for diagnosing a subject suffering from pancreatic cancer, or a pre-disposition thereto, or for providing a prognosis of the subject's condition, the apparatus comprising:-
(i) means for determining the concentration of a signature compound in a breath sample from a test subject; and
(ii) a reference for the concentration of the signature compound in a breath sample from an individual who does not suffer from pancreatic cancer,
wherein the apparatus is used to identify: a decrease in the concentration of the signature compound selected from a C₁-C₂₀ alkane in the breath sample from the test subject, compared to the reference, thereby suggesting that the subject suffers from pancreatic cancer, or has a pre-disposition thereto, or provides a negative prognosis of the subject's condition.

12. Use of an apparatus for determining the efficacy of treating a subject suffering from pancreatic cancer with a therapeutic agent or a specialised diet, the apparatus comprising:-
(a) means for determining the concentration of a signature compound in a breath sample from a test subject; and
(b) a reference for the concentration of the signature compound in a breath sample from an individual who does not suffer from pancreatic cancer,
wherein the apparatus is used to identify:
(i) an increase in the concentration of the signature compound selected from a C₁-C₂₀ alkane in the breath sample from the test subject, compared to the reference, thereby suggesting that the treatment regime with the therapeutic agent or the specialised diet is effective; or
(ii) a decrease in the concentration of the signature compound selected from a C₁-C₂₀ alkane in the breath sample from the test subject, compared to the reference, thereby suggesting that the treatment regime with the therapeutic agent or the specialised diet is ineffective.

13. Use of an apparatus according to either claim 11 or claim 12, wherein the concentration of an additional signature compound is determined, wherein the additional signature compound is as defined in any one of claims 6-10.

14. *In vitro* use of a signature compound selected from the group consisting of a C₁-C₂₀ alkane as a biomarker for diagnosing a subject suffering from pancreatic cancer, or a pre-disposition thereto, or for providing a prognosis of the subject's condition, wherein the signature compound is detected in a breath sample.

15. Use according to claim 14, wherein an additional signature compound is used, wherein the additional signature compound is as defined in any one of claims 6-10.

## Patentansprüche

1. Verfahren zum Diagnostizieren eines Subjekts, das an Bauchspeicheldrüsenkrebs leidet, oder einer Prädisposition dafür oder zum Bereitstellen einer Prognose des Zustands des Subjekts, wobei das Verfahren Analysieren der Konzentration einer Signaturverbindung in einer Atemprobe von einem Testsubjekt und Vergleichen dieser Konzentration mit einer Referenz für die Konzentration der Signaturverbindung in einem Individuum, das nicht an Bauchspeicheldrüsenkrebs leidet, umfasst, wobei eine Abnahme der Konzentration der Signaturverbindung ausgewählt aus einem C₁-C₂₀-Alkan in der Atemprobe von dem Testsubjekt verglichen mit der Referenz darauf hindeutet, dass das Subjekt an Bauchspeicheldrüsenkrebs leidet oder eine Prädisposition dafür aufweist, oder eine negative Prognose des Zustands des Subjekts bereitstellt.

2. Verfahren zum Bestimmen der Wirksamkeit des Behandelns eines Subjekts, das an Bauchspeicheldrüsenkrebs leidet, mit einem therapeutischen Mittel oder einer spezialisierten Diät, wobei das Verfahren Analysieren der Konzentration einer Signaturverbindung in einer Atemprobe von einem Testsubjekt und Vergleichen dieser Konzentration mit einer Referenz für die Konzentration der Signaturverbindung in einem Individuum, das nicht an Bauchspeicheldrüsenkrebs leidet, umfasst, wobei eine Erhöhung der Konzentration der Signaturverbindung ausgewählt aus einem C₁-C₂₀-Alkan in der Atemprobe von dem Testsubjekt verglichen mit der Referenz darauf hindeutet, dass das Behandlungsregime mit dem therapeutischen Mittel oder der spezialisierten Diät wirksam ist, oder wobei eine Abnahme der Konzentration der Signaturverbindung ausgewählt aus einem C₁-C₂₀-Alkan in der Atemprobe von dem Testsubjekt verglichen mit der Referenz darauf hindeutet, dass das Behandlungsregime mit dem therapeutischen Mittel oder der spezialisierten Diät unwirksam ist.

3. Verfahren nach Anspruch 1 oder Anspruch 2, wobei die Signaturverbindung ein C₅-Alkan ist.

4. Verfahren nach Anspruch 1 oder Anspruch 2, wobei die Signaturverbindung ein C₆-Alkan ist.

5. Verfahren nach Anspruch 1 oder Anspruch 2, wobei die Signaturverbindung ein C₁₄-Alkan ist.

6. Verfahren nach einem der Ansprüche 1, 3, 4 oder 5, wobei (i) eine Erhöhung der Konzentration einer zusätzlichen Signaturverbindung ausgewählt aus einem C₁-C₃-Aldehyd, einem C₁-C₃-Alkohol und einem C₂-C₁₀-Alkan, wobei ein erstes Kohlenstoffatom des C₂-C₁₀-Alkans mit einer =O-Gruppe substituiert ist und ein zweites Kohlenstoffatom mit einer -OH-Gruppe substituiert ist, in der Atemprobe von dem Testsubjekt, oder (ii) eine Abnahme der Konzentration einer zusätzlichen Signaturverbindung ausgewählt aus einem C₄-C₁₀-Alkohol, einer C₁-C₆-Carbonsäure und einem C₄-C₂₀-Aldehyd in der Atemprobe von dem Testsubjekt verglichen mit der Referenz darauf hindeutet, dass das Subjekt an Bauchspeicheldrüsenkrebs leidet oder eine Prädisposition dafür aufweist, oder eine negative Prognose des Zustands des Subjekts bereitstellt.

7. Verfahren nach einem der Ansprüche 2 bis 5, wobei (i) eine Abnahme der Konzentration einer zusätzlichen Signaturverbindung ausgewählt aus C₁-C₃-Aldehyd, C₁-C₃-Alkohol und C₂-C₁₀-Alkan, wobei ein erstes Kohlenstoffatom des C₂-C₁₀-Alkans mit einer =O-Gruppe substituiert ist und ein zweites Kohlenstoffatom mit einer -OH-Gruppe substituiert ist, in der Atemprobe von dem Testsubjekt verglichen mit der Referenz, oder (ii) eine Erhöhung der Konzentration einer zusätzlichen Signaturverbindung ausgewählt aus einem C₄-C₁₀-Alkohol, einer C₁-C₆-Carbonsäure und einem C₄-C₂₀-Aldehyd in der Atemprobe von dem Testsubjekt verglichen mit der Referenz darauf hindeutet, dass das Behandlungsregime mit dem therapeutischen Mittel oder der spezialisierten Diät wirksam ist, oder wobei (i) eine Erhöhung der Konzentration einer zusätzlichen Signaturverbindung ausgewählt aus einem C₁-C₃-Aldehyd, einem C₁-C₃-Alkohol und einem C₂-C₁₀-Alkan, wobei ein erstes Kohlenstoffatom des C₂-C₁₀-Alkans mit einer =O-Gruppe substituiert ist und ein zweites Kohlenstoffatom mit einer -OH-Gruppe substituiert ist, in der Atemprobe von dem Testsubjekt verglichen mit der Referenz, oder (ii) eine Abnahme der Konzentration einer zusätzlichen Signaturverbindung ausgewählt aus einem C₄-C₁₀-Alkohol, einer C₁-C₆-Carbonsäure und einem C₄-C₂₀-Aldehyd in der Atemprobe von dem Testsubjekt verglichen mit der Referenz darauf hindeutet, dass das Behandlungsregime mit dem therapeutischen Mittel oder der spezialisierten Diät unwirksam ist.

8. Verfahren nach Anspruch 6 oder Anspruch 7, wobei, wenn die zusätzliche Signaturverbindung ein C₁-C₃-Aldehyd ist, die Verbindung ein C₁-Aldehyd ist, und/oder
wobei, wenn die zusätzliche Signaturverbindung ein C₁-C₃-Alkohol ist, die Verbindung ein C₁-Alkohol oder ein C₃-Alkohol ist.

9. Verfahren nach einem der Ansprüche 5 bis 8, wobei, wenn die zusätzliche Signaturverbindung ein C₂-C₁₀-Alkan ist, wobei ein erstes Kohlenstoffatom mit der =O-Gruppe substituiert ist und ein zweites Kohlenstoffatom mit einer -OH-Gruppe substituiert ist, das Kohlenstoffatom substituiert mit der =O-Gruppe kein endständiges Kohlenstoffatom ist, und/oder
wobei, wenn die zusätzliche Signaturverbindung ein C₂-C₁₀-Alkan ist, wobei ein erstes Kohlenstoffatom mit der =O-Gruppe substituiert ist und ein zweites Kohlenstoffatom mit einer - OH-Gruppe substituiert ist, die Verbindung ein C₃-C₆-Alkan oder ein C₄-Alkan ist, wobei ein erstes Kohlenstoffatom mit der =O-Gruppe substituiert ist und ein zweites Kohlenstoffatom mit einer -OH-Gruppe substituiert ist.

10. Verfahren nach einem der Ansprüche 5 bis 9, wobei:
(i) wenn die zusätzliche Signaturverbindung ein C₄-C₁₀-Alkohol ist, die Verbindung ein C₄-C₇-Alkohol oder ein C₄-Alkohol ist;
(ii) wenn die zusätzliche Signaturverbindung eine C₁-C₆-Carbonsäure ist, die Verbindung eine C₂-C₄-Carbonsäure oder eine C₃-Carbonsäure ist;
(iii) wenn die zusätzliche Signaturverbindung ein C₄-C₂₀-Aldehyd ist, die Verbindung ein C₅-C₁₅-Aldehyd oder C₇-C₁₃-Aldehyd ist; und/oder
(ii) wenn die zusätzliche Signaturverbindung ein C₄-C₂₀-Aldehyd ist, die Verbindung ein C₈-Aldehyd oder ein C₉-Aldehyd oder ein C₁₀-Aldehyd oder ein C₁₁-Aldehyd ist.

11. Verwendung einer Vorrichtung zum Diagnostizieren eines Subjekts, das an Bauchspeicheldrüsenkrebs leidet, oder einer Prädisposition dafür oder zum Bereitstellen einer Prognose des Zustands des Subjekts, wobei die Vorrichtung Folgendes umfasst:-
(i) Mittel zum Bestimmen der Konzentration einer Signaturverbindung in einer Atemprobe von einem Testsubjekt; und
(ii) eine Referenz für die Konzentration der Signaturverbindung in einer Atemprobe von einem Individuum, das nicht an Bauchspeicheldrüsenkrebs leidet,
wobei die Vorrichtung verwendet wird, um Folgendes zu identifizieren: eine Abnahme der Konzentration der Signaturverbindung ausgewählt aus einem C₁-C₂₀-Alkan in der Atemprobe von dem Testsubjekt verglichen mit der Referenz, was darauf hindeutet, dass das Subjekt an Bauchspeicheldrüsenkrebs leidet oder eine Prädisposition dafür aufweist, oder eine negative Prognose des Zustands des Subjekts bereitstellt.

12. Verwendung einer Vorrichtung zum Bestimmen der Wirksamkeit des Behandelns eines Subjekts, das an Bauchspeicheldrüsenkrebs leidet, mit einem therapeutischen Mittel oder einer spezialisierten Diät, wobei die Vorrichtung Folgendes umfasst:-
(a) Mittel zum Bestimmen der Konzentration einer Signaturverbindung in einer Atemprobe von einem Testsubjekt; und
(b) eine Referenz für die Konzentration der Signaturverbindung in einer Atemprobe von einem Individuum, das nicht an Bauchspeicheldrüsenkrebs leidet,
wobei die Vorrichtung verwendet wird, um Folgendes zu identifizieren:
(i) eine Erhöhung der Konzentration der Signaturverbindung ausgewählt aus einem C₁-C₂₀-Alkan in der Atemprobe von dem Testsubjekt verglichen mit der Referenz, was darauf hindeutet, dass das Behandlungsregime mit dem therapeutischen Mittel oder der spezialisierten Diät wirksam ist; oder
(ii) eine Abnahme der Konzentration der Signaturverbindung ausgewählt aus einem C₁-C₂₀-Alkan in der Atemprobe von dem Testsubjekt verglichen mit der Referenz, was darauf hindeutet, dass das Behandlungsregime mit dem therapeutischen Mittel oder der spezialisierten Diät unwirksam ist.

13. Verwendung einer Vorrichtung nach Anspruch 11 oder Anspruch 12, wobei die Konzentration einer zusätzlichen Signaturverbindung bestimmt wird, wobei die zusätzliche Signaturverbindung wie in einem der Ansprüche 6-10 definiert ist.

14. In-vitro-Verwendung einer Signaturverbindung ausgewählt aus der Gruppe bestehend aus einem C₁-C₂₀-Alkan als Biomarker zum Diagnostizieren eines Subjekts, das an Bauchspeicheldrüsenkrebs leidet, oder einer Prädisposition dafür oder zum Bereitstellen einer Prognose des Zustands des Subjekts, wobei die Signaturverbindung in einer Atemprobe detektiert wird.

15. Verwendung nach Anspruch 14, wobei eine zusätzliche Signaturverbindung verwendet wird, wobei die zusätzliche Signaturverbindung wie in einem der Ansprüche 6-10 definiert ist.

## Revendications

1. Procédé permettant de diagnostiquer un sujet atteint d'un cancer du pancréas, ou présentant une prédisposition à celui-ci, ou permettant d'établir un pronostic de l'état du sujet, le procédé comprenant l'analyse de la concentration d'un composé signature dans un échantillon d'haleine provenant d'un sujet testé et la comparaison de cette concentration à une référence pour la concentration du composé signature chez un individu qui n'est pas atteint d'un cancer du pancréas, dans lequel une diminution de la concentration du composé signature choisi parmi un alcane en C₁-C₂₀ dans l'échantillon d'haleine provenant du sujet testé, par rapport à la référence, suggère que le sujet est atteint d'un cancer du pancréas, ou présente une prédisposition à celui-ci, ou établit un pronostic négatif de l'état du sujet.

2. Procédé permettant de déterminer l'efficacité du traitement d'un sujet atteint d'un cancer du pancréas par un agent thérapeutique ou un régime alimentaire spécialisé, le procédé comprenant l'analyse de la concentration d'un composé signature dans un échantillon d'haleine provenant d'un sujet testé et la comparaison de cette concentration à une référence pour la concentration du composé signature chez un individu qui n'est pas atteint d'un cancer du pancréas, dans lequel une augmentation de la concentration du composé signature choisi parmi un alcane en C₁-C₂₀ dans l'échantillon d'haleine provenant du sujet testé, par rapport à la référence, suggère que le régime de traitement par l'agent thérapeutique ou le régime alimentaire spécialisé est efficace, ou dans lequel une diminution de la concentration du composé signature choisi parmi un alcane en C₁-C₂₀ dans l'échantillon d'haleine provenant du sujet testé, par rapport à la référence, suggère que le régime de traitement par l'agent thérapeutique ou le régime alimentaire spécialisé est inefficace.

3. Procédé selon la revendication 1 ou la revendication 2, dans lequel le composé signature est un alcane en C₅.

4. Procédé selon la revendication 1 ou la revendication 2, dans lequel le composé signature est un alcane en C₆.

5. Procédé selon la revendication 1 ou la revendication 2, dans lequel le composé signature est un alcane en C₁₄.

6. Procédé selon l'une quelconque des revendications 1, 3, 4 ou 5, dans lequel (i) une augmentation de la concentration d'un composé signature supplémentaire choisi parmi un aldéhyde en C₁-C₃, un alcool en C₁-C₃ et un alcane en C₂-C₁₀, dans lequel un premier atome de carbone de l'alcane en C₂-C₁₀ est substitué par un groupe =O et un second atome de carbone est substitué par un groupe -OH, dans l'échantillon d'haleine provenant du sujet testé, ou (ii) une diminution de la concentration d'un composé signature supplémentaire choisi parmi un alcool en C₄-C₁₀, un acide carboxylique en C₁-C₆ et un aldéhyde en C₄-C₂₀, dans l'échantillon d'haleine provenant du sujet testé, par rapport à la référence, suggère que le sujet est atteint d'un cancer du pancréas, ou présente une prédisposition à celui-ci, ou fournit un pronostic négatif de l'état du sujet.

7. Procédé selon l'une quelconque des revendications 2 à 5, dans lequel (i) une diminution de la concentration d'un composé signature supplémentaire choisi parmi un aldéhyde en C₁-C₃, un alcool en C₁-C₃ et un alcane en C₂-C₁₀, dans lequel un premier atome de carbone de l'alcane en C₂-C₁₀ est substitué par un groupe =O et un second atome de carbone est substitué par un groupe -OH, dans l'échantillon d'haleine provenant du sujet testé, par rapport à la référence, ou (ii) une augmentation de la concentration d'un composé signature supplémentaire choisi parmi un alcool en C₄-C₁₀, un acide carboxylique en C₁-C₆ et un aldéhyde en C₄-C₂₀, dans l'échantillon d'haleine provenant du sujet testé, par rapport à la référence, suggère que le régime de traitement par l'agent thérapeutique ou le régime alimentaire spécialisé est efficace, ou dans lequel (i) une augmentation de la concentration d'un composé signature supplémentaire choisi parmi un aldéhyde en C₁-C₃, un alcool en C₁-C₃ et un alcane en C₂-C₁₀, dans lequel un premier atome de carbone de l'alcane en C₂-C₁₀ est substitué par un groupe =O et un second atome de carbone est substitué par un groupe -OH, dans l'échantillon d'haleine provenant du sujet testé, par rapport à la référence, ou (ii) une diminution de la concentration d'un composé signature supplémentaire choisi parmi un alcool en C₄-C₁₀, un acide carboxylique en C₁-C₆ et un aldéhyde en C₄-C₂₀, dans l'échantillon d'haleine provenant du sujet testé, par rapport à la référence, suggère que le régime de traitement par l'agent thérapeutique ou le régime alimentaire spécialisé est inefficace.

8. Procédé selon la revendication 6 ou la revendication 7, dans lequel, lorsque le composé signature supplémentaire est un aldéhyde en C₁-C₃, le composé est un aldéhyde en C₁, et/ou
dans lequel, lorsque le composé signature supplémentaire est un alcool en C₁-C₃, le composé est un alcool en C₁ ou un alcool en C₃.

9. Procédé selon l'une quelconque des revendications 5 à 8, dans lequel, lorsque le composé signature supplémentaire est un alcane en C₂-C₁₀, dans lequel un premier atome de carbone est substitué par le groupe =O et un second atome de carbone est substitué par un groupe -OH, l'atome de carbone substitué par le groupe =O n'est pas un atome de carbone terminal, et/ou
dans lequel, lorsque le composé signature supplémentaire est un alcane en C₂-C₁₀, dans lequel un premier atome de carbone est substitué par le groupe =O et un second atome de carbone est substitué par un groupe -OH, le composé est un alcane en C₃-C₆ ou un alcane en C₄, dans lequel un premier atome de carbone est substitué par le groupe =O et un second atome de carbone est substitué par un groupe -OH.

10. Procédé selon l'une quelconque des revendications 5 à 9, dans lequel :
(i) lorsque le composé signature supplémentaire est un alcool en C₄-C₁₀, le composé est un alcool en C₄-C₇ ou un alcool en C₄ ;
(ii) lorsque le composé signature supplémentaire est un acide carboxylique en C₁-C₆, le composé est un acide carboxylique en C₂-C₄ ou un acide carboxylique en C₃ ;
(iii) lorsque le composé signature supplémentaire est un aldéhyde en C₄-C₂₀, le composé est un aldéhyde en C₅-C₁₅ ou un aldéhyde en C₇-C₁₃ ; et/ou
(ii) lorsque le composé signature supplémentaire est un aldéhyde en C₄-C₂₀, le composé est un aldéhyde en C₈, ou un aldéhyde en C₉, ou un aldéhyde en C₁₀, ou un aldéhyde en C₁₁.

11. Utilisation d'un appareil permettant de diagnostiquer un sujet atteint d'un cancer du pancréas, ou présentant une prédisposition à celui-ci, ou permettant d'établir un pronostic de l'état du sujet, l'appareil comprenant :
(i) un moyen permettant de déterminer la concentration d'un composé signature dans un échantillon d'haleine provenant d'un sujet testé ; et
(ii) une référence pour la concentration du composé signature dans un échantillon d'haleine provenant d'un individu qui n'est pas atteint d'un cancer du pancréas,
ledit appareil étant utilisé pour identifier : une diminution de la concentration du composé signature choisi parmi un alcane en C₁-C₂₀ dans l'échantillon d'haleine provenant du sujet testé, par rapport à la référence, suggérant ainsi que le sujet est atteint d'un cancer du pancréas, ou présente une prédisposition à celui-ci, ou établissant un pronostic négatif de l'état du sujet.

12. Utilisation d'un appareil permettant de déterminer l'efficacité du traitement d'un sujet atteint d'un cancer du pancréas par un agent thérapeutique ou un régime alimentaire spécialisé, l'appareil comprenant :
(a) un moyen permettant de déterminer la concentration d'un composé signature dans un échantillon d'haleine provenant d'un sujet testé ; et
(b) une référence pour la concentration du composé signature dans un échantillon d'haleine provenant d'un individu qui n'est pas atteint d'un cancer du pancréas,
ledit appareil étant utilisé pour identifier :
(i) une augmentation de la concentration du composé signature choisi parmi un alcane en C₁-C₂₀ dans l'échantillon d'haleine provenant du sujet testé, par rapport à la référence, suggérant ainsi que le régime de traitement par l'agent thérapeutique ou le régime alimentaire spécialisé est efficace ; ou
(ii) une diminution de la concentration du composé signature choisi parmi un alcane en C₁-C₂₀ dans l'échantillon d'haleine du sujet testé, par rapport à la référence, suggérant ainsi que le régime de traitement par l'agent thérapeutique ou le régime alimentaire spécialisé est inefficace.

13. Utilisation d'un appareil selon la revendication 11 ou la revendication 12, dans laquelle la concentration d'un composé signature supplémentaire est déterminée, ledit composé signature supplémentaire étant tel que défini dans l'une quelconque des revendications 6 à 10.

14. Utilisation *in vitro* d'un composé signature choisi dans le groupe constitué par un alcane en C₁-C₂₀ en tant que biomarqueur pour diagnostiquer un sujet atteint d'un cancer du pancréas, ou présentant une prédisposition à celui-ci, ou pour établir un pronostic de l'état du sujet, ledit composé signature étant détecté dans un échantillon d'haleine.

15. Utilisation selon la revendication 14, dans laquelle un composé signature supplémentaire est utilisé, ledit composé signature supplémentaire étant tel que défini dans l'une quelconque des revendications 6 à 10.
